(19) Europäisches Patentamt
European Patent Office
Office européen des brevets

(11) EP 1 763 515 B1

(12) EUROPÄISCHE PATENTSCHRIFT

(45) Veröffentlichungstag und Bekanntmachung des Hinweises auf die Patenterteilung:
**12.03.2008 Patentblatt 2008/11**

(21) Anmeldenummer: **05750284.1**

(22) Anmeldetag: **15.06.2005**

(51) Int Cl.:
***C07D 217/26*** (2006.01) ***C07D 493/04*** (2006.01)
***C07D 223/16*** (2006.01) ***A61K 31/55*** (2006.01)
***A61P 19/00*** (2006.01)

(86) Internationale Anmeldenummer:
**PCT/EP2005/006416**

(87) Internationale Veröffentlichungsnummer:
**WO 2006/002764 (12.01.2006 Gazette 2006/02)**

(54) **4-TRIFLUORMETHOXYPHENOXYBENZOL-4'-SULFONSÄUREN, VERFAHREN ZU IHRER HERSTELLUNG UND VERWENDUNG IN ARZNEIMITTELN**

4-TRIFLUOROMETHOXYPHENOXYBENZOL-4'-SULFONIC ACIDS, METHOD FOR THE PRODUCTION AND USE THEREOF IN MEDICAMENTS

ACIDES 4-TRIFLUOROMETHOXYPHENOXYBENZENE-4'-SULFONIQUES, LEUR PROCEDE DE PRODUCTION ET LEUR UTILISATION DANS DES MEDICAMENTS

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **30.06.2004 DE 102004031620**

(43) Veröffentlichungstag der Anmeldung:
**21.03.2007 Patentblatt 2007/12**

(73) Patentinhaber: **Sanofi-Aventis Deutschland GmbH**
**65929 Frankfurt am Main (DE)**

(72) Erfinder:
- **SCHUDOK, Manfred**
  **65817 Eppstein (DE)**
- **HOFMEISTER, Armin**
  **55278 Dexheim (DE)**

(56) Entgegenhaltungen:
**WO-A-97/18194** **WO-A-20/05030728**

- **CAROL K. WADA ET AL.: "Phenoxyphenyl Sulfone N-Formylhydroxylamines (Retrohydroxamates) as Potent, Selective, Orally Bioavilable Matrix Metalloproteinase Inhibitors" J. MED. CHEM., Bd. 45, 2002, Seiten 219-232, XP002344552**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 763 515 B1

## Beschreibung

Die Erfindung betrifft neue Derivate von 4-Trifluormethoxyphenoxybenzol wie der 4-Trifluor-methoxyphenoxybenzol-4'-sulfonsäure, das respektive Sulfonsäurechlorid, Derivate wie Sulfonamide sowie Verfahren zu ihrer Herstellung und Verwendung derselben als Arzneimittel.

Pharmakologisch aktive Substanzen sind häufig aus einem oder mehreren Ringsystemen aufgebaut. Hierbei kann es sich um gesättigte oder ungesättigte Carbo-oder Heterocyclen handeln. Zur Ausübung der biologischen Aktivität ist eine bestimmte räumliche Anordnung erforderlich. Darüber hinaus gibt es eine ganze Reihe weiterer unterschiedlicher aber sehr wichtiger Wechselwirkungen, die zu einer Bindungsaffinität beitragen. Beispielsweise kann es sich um Pi-Pi-Wechselwirkungen aromatischer Systeme zwischen Protein und Inhibitor handeln, um ionische Wechselwirkungen, oder um Säure-Base-Wechselwirkungen. Für letztere sind insbesondere funktionelle Gruppen verantwortlich. Diese sind oftmals an oben erwähnten Ringsystemen "angebracht". Jedoch ist die biologische Aktivität nur ein Teilaspekt, der von Wirkstoffen, die als potentielle Arzneimittel entwickelt werden sollen, erfüllt werden muss. Ein anderer wichtiger Bereich, der in der Vergangenheit oftmals unterschätzt wurde, ist in der Absorption, der Distribution, dem Metabolismus und der Ausscheidung des Wirkstoffes zu sehen. Für das unterschiedliche Verhalten der Moleküle in diesem Bereich sind genauso wie für die biologische Aktivität oftmals einzelne Teile des Moleküls in besonderem Maße verantwortlich. Es kann sich auch hier um Ringsysteme, deren besondere Eigenschaften und funktionelle Gruppen handeln. Allerdings ist eine Korrelation mit beispielsweise bestimmten physikochemischen Eigenschaften dieser Gruppen in vielen Fällen nicht befriedigend möglich oder eine Vorhersage durch Methoden der Computational Chemistry, im Gegensatz zu dem Bereich der biologischen Aktivität, bislang nur andeutungsweise möglich. Besondere Komplexität zeigt sich dann, wenn kleine Modifikationen an diesen funktionellen Gruppen vorgenommen werden und zu sehr starken Effekten führen. Hiermit ist gemeint, dass sich beispielsweise Absorption, Distribution (gleich Verteilung), Metabolismus und Ausscheidung signifikant verändern können. So kann durchaus aus einer Leitstruktur mit ungenügenden Eigenschaften ein Entwicklungskandidat werden.

Überraschenderweise wurde nun gefunden, dass Verbindungen, die erfindungsgemäßen Reste tragen, deutlich bessere pharmakokinetische Eigenschaften besitzen als sehr eng verwandte Verbindungen, die einfache Alkylether- oder Alkylfluorid-Seitenketten besitzen. Verbesserte pharmakokinetische Eigenschaften heißt hier, dass von der Beobachtung her sowohl maximal erreichbare Plasmaspiegel höher sind, als auch Halbwertszeiten verlängert sind. Das heißt, dass also eine positive Beeinflussung besonders von Absorption, Metabolismus und Ausscheidung stattfindet.
Gleichzeitig sind beispielsweise die häufig in Wirkstoffen zu findenden erfindungsgemäßen Sulfonamide, die aus den bislang unbekannten erfindungsgemäßen Sulfonylchloriden hergestellt werden, neu. Gleiches gilt für die korrespondierende Sulfonsäuren.

Die erfindungsgemäßen Verbindungen können breit eingesetzt werden. Beispielsweise tragen Matrix-Metalloproteinase-Inhibitoren (MMP-) häufig zum erfindungsgemäßen Typ ähnliche Seitenketten. Cyclische und insbesondere bicyclische Grundgerüste sind breit beschrieben. Beispielsweise werden in WO9718194 Tetrahydroisochinolin-Derivate beschrieben und in WO03/016248 werden weitere Heterocyclen beschrieben.

Die Erfindung betrifft daher eine Verbindung der Formel I

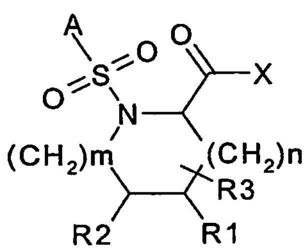 (I)

und/oder alle stereoisomeren Formen der Verbindung der Formel I und/oder Gemische dieser Formen in jedem Verhältnis, und/oder ein physiologisch verträgliches Salz der Verbindung der Formel I, wobei
X für -OH oder-NH-OH steht,
A für einen Rest der Formel II steht,

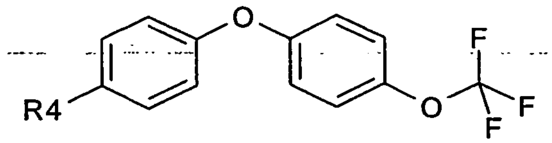

(II)

worin R4 die kovalente Bindung mit dem S-Atom der Formel I bedeutet,

R1, R2 und R3 gleich oder verschieden sind und unabhängig voneinander für

1) Wasserstoffatom,
2) -($C_1$-$C_6$)-Alkyl, worin Alkyl unsubstituiert oder ein- oder zweifach durch - ($C_3$-$C_6$)-Cycloalkyl, -($C_2$-$C_4$)-Alkenylen, -($C_2$-$C_6$)-Alkinyl, -($C_6$-$C_{14}$)-Aryl oder Het-Ring substituiert ist,
3) -C(O)-O-R8, worin R8

3)1) Wasserstoffatom,
3)2) -($C_1$-$C_6$)-Alkyl, worin Alkyl unsubstituiert oder ein- oder zweifach durch -($C_3$-$C_6$)-Cycloalkyl, -($C_2$-$C_4$)-Alkenylen, -($C_2$-$C_6$)-Alkinyl, -($C_6$-$C_{14}$)-Aryl, oder Het-Ring oder ein- bis fünffach durch Fluor, substituiert ist,
3)3) -($C_6$-$C_{14}$)-Aryl oder
3)4) Het-Ring bedeutet,

4) -O-R8, worin R8 die oben genannte Bedeutung hat,
5) -($C_3$-$C_6$)-Cycloalkyl,
6) -Halogen,
7) -$NO_2$, oder
8) -CN, stehen, oder
9) R1 und R2 bilden zusammen mit den Kohlenstoffatomen an die sie gebunden sind einen -($C_6$-$C_{14}$)-Aryl-Ring, worin der Ring unsubstituiert oder ein- oder zweifach durch G substituiert ist,
10) R1 und R2 bilden zusammen mit den Kohlenstoffatomen an die sie gebunden sind einen -($C_5$-$C_7$)-Cycloalkyl-Ring, worin der Ring unsubstituiert oder ein- oder zweifach durch G substituiert ist, oder
11) R1 und R2 bilden zusammen mit den Kohlenstoffatomen, an die sie gebunden sind, einen 5-, 6- oder 7-gliedrigen Het-Ring, wobei der Ring unsubstituiert oder einfach durch G substituiert ist, oder
12) R1 und R2 bilden zusammen mit den Kohlenstoffatomen, an die sie gebunden sind, ein Indolyl, worin das Indolyl unsubstituiert oder ein- oder zweifach durch G substituiert ist,

G für

1) Wasserstoffatom,
2) Halogen,
3) =O,
4) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_4$)-Alkenylen, -($C_2$-$C_6$)-Alkinyl, -($C_6$-$C_{14}$)-Aryl oder Het-Ring substituiert ist,
5) -($C_6$-$C_{14}$)-Aryl,
6) Het-Ring,
7) -C(O)-O-R10, worin R10

a) -($C_1$-$C_6$)-Alkyl, worin Alkyl unsubstituiert oder ein- oder zweifach durch -($C_3$-$C_6$)-Cycloalkyl, -($C_2$-$C_4$)-Alkenylen, - ($C_2$-$C_6$)-Alkinyl,
-($C_6$-$C_{14}$)-Aryl oder Het-Ring substituiert ist,
b) -($C_6$-$C_{14}$)-Aryl oder
c) Het-Ring, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl

oder Het-Ring substituiert ist, oder
b) -($C_6$-$C_{14}$)-Aryl oder
c) Het-Ring, bedeutet,

10) -C(S)-NH-R11, worin R11 wie oben definiert ist, bedeutet,
11) -O-R12, worin R12

a) Wasserstoffatom,
b) -($C_1$-$C_6$)-Alkyl, worin Alkyl unsubstituiert oder ein-, zwei-oder dreifach durch Halogen, -($C_3$-$C_6$)-Cycloalkyl, -($C_2$-$C_4$)-Alkenylen,
-($C_2$-$C_6$)-Alkinyl, -($C_6$-$C_{14}$)-Aryl oder Het-Ring substituiert ist,
c) -($C_6$-$C_{14}$)-Aryl,
d) Het-Ring,
e) -C(O)-O-R13, worin R13

e)1) -($C_1$-$C_6$)-Alkyl, worin Alkyl unsubstituiert oder ein-oder zweifach durch -($C_3$-$C_6$)-Cycloalkyl, -($C_2$-$C_4$)-Alkenylen,
-($C_2$-$C_6$)-Alkinyl, -($C_6$-$C_{14}$)-Aryl, oder Het-Ring substituiert ist, oder
e)2) -($C_6$-$C_{14}$)-Aryl oder
e)3) Het-Ring, bedeutet,

f) -C(S)-O-R13, worin R13 wie oben definiert ist,
g) -C(O)-NH-R14, worin R14

g)1) -($C_1$-$C_6$)-Alkyl, worin Alkyl unsubstituiert oder ein-oder zweifach durch -($C_3$-$C_6$)-Cycloalkyl, -($C_2$-$C_4$)-Alkenylen,
-($C_2$-$C_6$)-Alkinyl, -($C_6$-$C_{14}$)-Aryl oder Het-Ring substituiert ist, oder
g)2) -($C_6$-$C_{14}$)-Aryl oder
g)3) Het-Ring bedeutet, oder

h) -C(S)-NH-R14, worin R14 wie oben definiert ist, bedeutet,

12) -C(O)-R10, worin R10 wie oben definiert ist,
13) -S(O)p-R12, worin R12 wie oben definiert ist und p die ganzen Zahlen Null, 1 oder 2 bedeutet,
14) -$NO_2$,
15) -CN oder
16) -N(R15)-R12, worin R15

16)1) Wasserstoffatom,
16)2) -($C_1$-$C_6$)-Alkyl oder
16)3) -$SO_2$-($C_1$-$C_6$)-Alkyl, worin Alkyl unsubstituiert oder ein- oder zweifach durch -($C_3$-$C_6$)-Cycloalkyl, -($C_2$-$C_4$)-Alkenylen, -($C_2$-$C_6$)-Alkinyl, -($C_6$-$C_{14}$)-Aryl oder Het-Ring substituiert ist, bedeutet und R12 wie oben definiert ist, oder

17) -$SO_2$-N(R12)-R16, worin R12 wie oben definiert ist und worin R16

17)1) Wasserstoffatom,
17)2) -($C_1$-$C_6$)-Alkyl, worin Alkyl unsubstituiert oder ein- oder zweifach durch -($C_3$-$C_6$)-Cycloalkyl, -($C_2$-$C_4$)-Alkenylen, -($C_2$-$C_6$)-Alkinyl, - ($C_6$-$C_{14}$)-Aryl oder Het-Ring substituiert ist,
17)3) -C(O)-O-R8, worin R8 die oben genannte Bedeutung hat,
17)4) -O-R8, worin R8 die oben genannte Bedeutung hat, oder
17)5) -($C_3$-$C_6$)-Cycloalkyl bedeutet, steht, und

m und n gleich oder verschieden sind und für die Zahlen Null, 1, 2 oder 3 stehen, mit der Maßgabe, dass die Summe von m und n den Betrag Null, 1, 2 oder 3 hat.

Ein weiterer Gegenstand der Erfindung ist die Verbindung der Formel I, wobei
X für -OH oder-NH-OH steht,

A für einen Rest der Formel II steht,
R1 und R2 bilden zusammen mit den Kohlenstoffatomen an die sie gebunden sind

a) einen -($C_6$-$C_{14}$)-Aryl-Ring, worin Aryl ein Rest aus der Reihe Phenyl, Naphthyl, 1-Naphthyl, 2-Naphthyl, Anthryl oder Fluorenyl bedeutet und unsubstituiert oder ein- oder zweifach durch G substituiert ist, oder
b) einen 5-, 6- oder 7-gliedrigen Het-Ring, worin Het-Ring ein Rest aus der Reihe Furan, Imidazol, Isoxazol, Oxazol Pyrazin, Pyrazol, Pyridazin, Pyridin, Pyrimidin, Thiazol oder Thiophen bedeutet und unsubstituiert oder ein- oder zweifach durch G substituiert ist, oder
c) ein Indolyl, worin das Indolyl unsubstituiert oder ein- oder zweifach durch G substituiert ist,

G für

1) Wasserstoffatom,
2) Fluor, Chlor, Brom oder Jod,
3) =O,
4) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_6$)-Alkinyl, -($C_6$-$C_{14}$)-Aryl, worin Aryl für Phenyl oder Naphthyl steht, oder Het-Ring, worin der Het-Ring ein Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathünyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl ist, substituiert ist,
5) -($C_6$-$C_{14}$)-Aryl, worin Aryl für Phenyl oder Naphthyl steht,
6) Het-Ring, worin Het wie oben definiert ist,
7) -C(O)-O-R10, worin R10

a) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl, oder Het-Ring substituiert ist, worin Aryl für Phenyl oder Naphthyl steht und Het wie oben definiert ist,
c) -($C_6$-$C_{14}$)-Aryl, worin Aryl für Phenyl oder Naphthyl steht, oder
c) Het-Ring, worin Het wie oben definiert ist, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl oder Het-Ring substituiert ist, worin Aryl für Phenyl oder Naphthyl steht und Het wie oben definiert ist, oder
b) -($C_6$-$C_{14}$)-Aryl, worin Aryl für Phenyl oder Naphthyl steht, oder
c) Het-Ring, worin Het wie oben definiert ist, bedeutet,

10) -C(S)-NH-R11, worin R11 wie oben definiert ist, bedeutet,
11) -O-R12, worin R12

a) Wasserstoffatom,
b) -($C_1$-$C_6$)-Alkyl, worin Alkyl unsubstituiert oder ein-, zwei-oder dreifach durch Halogen, -(Cg-Cg)-Cycloalkyl, -($C_2$-$C_6$)-Alkinyl,

-($C_6$-$C_{14}$)-Aryl oder Het-Ring substituiert ist, worin Aryl für Phenyl oder Naphthyl steht und Het wie oben definiert ist,
c) -($C_6$-$C_{14}$)-Aryl, worin Aryl für Phenyl oder Naphthyl steht,
d) Het-Ring, worin Het wie oben definiert ist,
e) -C(O)-O-R13, worin R13

e)1) -($C_1$-$C_6$)-Alkyl, worin Alkyl unsubstituiert oder ein-oder zweifach durch -($C_3$-$C_6$)-Cycloalkyl, -($C_2$-$C_6$)-Alkinyl,
-($C_6$-$C_{14}$)-Aryl oder Het-Ring substituiert ist, worin Aryl für Phenyl oder Naphthyl steht und Het wie oben definiert ist,
e)2) -($C_6$-$C_{14}$)-Aryl, worin Aryl für Phenyl oder Naphthyl steht, oder
e)3) Het-Ring, worin Het wie oben definiert ist, bedeutet,

f) -C(S)-O-R13, worin R13 wie oben definiert ist,
g) -C(O)-NH-R14, worin R14

g)1) -($C_1$-$C_6$)-Alkyl, worin Alkyl unsubstituiert oder ein-oder zweifach durch -($C_3$-$C_6$)-Cycloalkyl, -($C_2$-$C_6$)-Alkinyl,
-($C_6$-$C_{14}$)-Aryl oder Het-Ring substituiert ist, worin Aryl für Phenyl oder Naphthyl steht und Het wie oben definiert ist,
g)2) -($C_6$-$C_{14}$)-Aryl, worin Aryl für Phenyl oder Naphthyl steht, oder
g)3) Het-Ring bedeutet, worin Het wie oben definiert ist, oder

h) -C(S)-NH-R14, worin R14 wie oben definiert ist, bedeutet,

12) -C(O)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN,
16) -N(R15)-R12, worin R15

16)1) Wasserstoffatom,
16)2) -($C_1$-$C_6$)-Alkyl oder
16)3) -$SO_2$- ($C_1$-$C_6$)-Alkyl worin Alkyl unsubstituiert oder ein-oder zweifach durch -($C_3$-$C_6$)-Cycloalkyl, -($C_2$-$C_6$)-Alkinyl, -($C_6$-$C_{14}$)-Aryl oder Het-Ring substituiert ist, worin Aryl für Phenyl oder Naphthyl steht und Het wie oben definiert ist, bedeutet und R12 wie oben definiert ist, oder

17) -$SO_2$-N(R12)-R16, worin R12 wie oben definiert ist und worin R16

17)1) Wasserstoffatom,
17)2) -($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, worin Aryl für Phenyl oder Naphthyl steht und Het wie oben definiert ist,
17)3) -C(O)-O-R8, worin R8 die oben genannte Bedeutung hat,
17)4) -O-R8, worin R8 die oben genannte Bedeutung hat, oder
17)5) -($C_3$-$C_6$)-Cycloalkyl bedeutet, steht, und

m für die Zahl 1 oder 2 steht und n für die Zahl Null steht,
m für die Zahl 1 steht und n für die Zahl zwei steht oder
m und n gleich sind und jeweils für die Zahl 1 stehen.

Ein weiterer Gegenstand der Erfindung ist die Verbindung der Formel I, wobei

X für -OH oder-NH-OH steht,
A für einen Rest der Formel II steht,
m für die Zahl 1 steht und n für die Zahl zwei steht oder
m und n gleich sind und jeweils für die Zahl 1 stehen und
R1 und R2 bilden zusammen mit den Kohlenstoffatomen an die sie gebunden sind ein Phenyl, Tetrahydrofuranyl

oder Cylohexyl.

Ein weiterer Gegenstand der Erfindung ist die Verbindung der Formel I aus der Reihe
2-[4-(4-Trifluormethoxy-phenoxy)-benzolsulfonyl]-1,2,3,4-tetrahydro-isochinolin-1-carbonsäure-hydroxyamid,
2-[4-(4-Trifluoromethoxyphenoxy)-benzolsulfonyl]-decahydro-isochinolin-1-(N-hydroxy)-carboxamid,
5-[4-(4-Trifluoromethoxy-phenoxy)-benzolsulfonyl]-octahydro-furo[3,2-c]pyridin-4-carbonsäure,
5-[4-(4-Trifluoromethoxy-phenoxy)-benzolsulfonyl]-octahydro-furo[3,2-c]pyridin-4-(N-hydroxy)-carboxamid oder
2-[4-(4-Trifluoromethoxy-phenoxy)-benzolsulfonyl]-decahydro-benzo[C]azepin-1-(N-hydroxy)-carboxamid.

Unter dem Begriff "($C_1$-$C_6$)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl.
Unter dem Begriff "-$(CH_2)_n$-, worin n die Zahl Null, 1, 2 oder 3 bedeutet" wird für n gleich Null eine kovalente Bindung, n gleich 1 der Rest Methylen, n gleich 2 der Rest Ethylen und n gleich 3 Propylen verstanden. Die Bedeutungen des Begriffs "$(CH_2)_m$-, worin m die Zahl Null, 1, 2 oder 3 bedeutet" sind analog zum Begriff -$(CH_2)_n$-. Unter dem Begriff "($C_2$-$C_4$)-Alkenylen," werden Kohlenwasserstoffreste verstanden, deren Kohlenstoffkette geradkettig oder verzweigt ist und 2 bis 4 Kohlenstoffatome enthält und je nach Kettenlänge 1 oder 2 Doppelbindungen aufweisen, beispielsweise Ethenylen, Propenylen, Iso-Propenylen, Iso-Butenylen oder Butenylen; die Substituenten an der Doppelbindung können, sofern die prinzipielle Möglichkeit besteht, E- oder Z-ständig angeordnet sein.
Unter dem Begriff "-($C_2$-$C_6$)-Alkinylen," werden Kohlenwasserstoffreste verstanden, deren Kohlenstoffkette geradkettig oder verzweigt ist und 2 bis 6 Kohlenstoffatome enthält und je nach Kettenlänge 1 oder 2 Dreifachbindungen aufweisen, beispielsweise Ethinylen, Propenylen, Iso-Propinylen, Iso-Buthylinylen, Butinylen, Pentinylen oder Isomere von Pentinylen oder Hexinylen oder Isomere von Hexinylen.

Unter dem Begriff "($C_3$-$C_6$)Cycloalkyl" werden Reste verstanden wie Verbindungen, die sich von 3- bis 6-gliedrige Monocyclen wie Cyclopropyl, Cyclobutyl, Cyclopentyl oder Cyclohexyl herleiten. Unter dem Begriff "($C_5$-$C_7$)-Cycloalkyl" werden Reste verstanden wie Verbindungen, die sich von 5- bis 7-gliedrige Monocyclen wie Cyclopentyl, Cyclohexyl oder Cyloseptyl herleiten.
Unter dem Begriff "-($C_6$-$C_{14}$)-Aryl" werden aromatische Kohlenstoffreste verstanden mit 6 bis 14 Kohlenstoffatomen im Ring. -($C_6$-$C_{14}$)-Arylreste sind beispielsweise Phenyl, Naphthyl, 1-Naphthyl, 2-Naphthyl, Anthryl oder Fluorenyl. Naphthylreste und insbesondere Phenylreste sind bevorzugte Arylreste.
Unter dem Begriff "Het-Ring" werden Ringsysteme verstanden mit 4 bis 15 Kohlenstoffatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, lmidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathünyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl.
Bevorzugte Het-Ringe sind die Reste Benzofuranyl, Benzimidazolyl, Benzoxazolyl, Benzothiazolyl, Benzothiophenyl, 1,3-Benzodioxolyl, Chinazolinyl, Chinolinyl, Chinoxalinyl, Chromanyl, Cinnolinyl, Furanyl; wie 2-Furanyl und 3-Furanyl; Imidazolyl, Indolyl, Indazolyl, Isochinolinyl, Isochromanyl, Isoindolyl, Isothiazolyl, Isoxazolyl, Oxazolyl, Phthalazinyl, Pteridinyl, Pyrazinyl, Pyrazolyl, Pyridazinyl, Pyridoimidazolyl, Pyridopyridinyl, Pyridopyrimidinyl, Pyridyl; wie 2-Pyridyl, 3-Pyridyl oder 4-Pyridyl; Pyrimidinyl, Pyrrolyl; wie 2-Pyrrolyl und 3-Pyrrolyl; Purinyl, Thiazolyl, Tetrazolyl oder Thienyl; wie 2-Thienyl und 3-Thienyl.
Unter dem Begriff "R1 und R2 bilden zusammen mit den Kohlenstoffatomen, an die sie gebunden sind, einen 5-, 6- oder 7-gliedrigen Het-Ring" werden Verbindungen verstanden die sich beispielsweise von den folgenden Verbindungen ableiten wie Dioxan, Furan, Imidazol, Imidazolin, Imidazolidin, Isothiazol,lisothiazolidin, Isothiazolin, Isoxazol, Isoxazolin, Isoxazolidin, 2-Isoxazoline, Morpholin, Piperazine, 1,2-Oxazin, 1,3-Oxazin, 1,4-Oxazin, Oxazol, Oxazolidin, Oxazolidon,

Piperazin, Piperidin, Pyran, Pyrazin, Pyrazol, Pyrazolin, Pyrazolidin, Pyridazin, Pyridin, Pyrimidin, Pyrrol, Pyrrolidin, Pyrrolidinon, Pyrrolin, Tetrahydrofuran, Tetrahydropyran, Tetrahydropyridin, Tetrazin, Tetrazol, Thiadiazin, 1,2-Thiazin, 1,3-Thiazin, 1,4-Thiazin, 1,3-Thiazol, Thiazol, Thiazolidin, Thiazolin, Thiophen, Thietan, Thiomorpholin, Thiopyran, 1,2,3-Triazin, 1,2,4-Triazin, 1,3,5-Triazin, 1,2,3-Triazol oder 1,2,4-Triazol. Unter dem Begriff "Halogen" wird Fluor, Chlor, Brom oder Jod verstanden.

Die Erfindung betrifft ferner ein Verfahren zur Herstellung der Verbindung der Formel I und/oder einer stereoisomeren Form der Verbindung der Formel und/oder eines physiologisch verträglichen Salzes der Verbindung der Formel I, das dadurch gekennzeichnet ist, dass man

a) eine Verbindung der Formel III

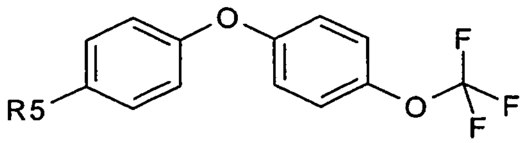

(III)

worin R5 für Wasserstoffatom, $NH_2$, Li, Mg. SH, S-$CH_3$, Cl, Br, I oder Si-$(CH_3)_3$ steht, in eine Verbindung der Formel IV überführt,

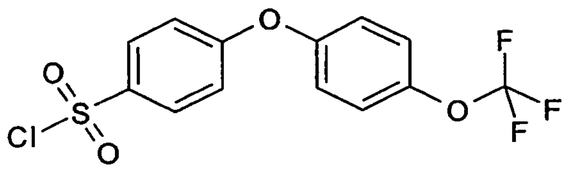

(IV)

und mit einer Verbindung der Formel V,

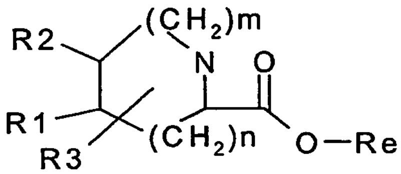

(V)

worin R1, R2, R3, m und n wie in Formel I definiert sind und Re ein Wasserstoffatom oder eine Ester-Schutzgruppe darstellt, in Gegenwart einer Base oder nach Silylierung mit einem geeigneten Silylierungsmittel zu einer Verbindung der Formel VI umsetzt,

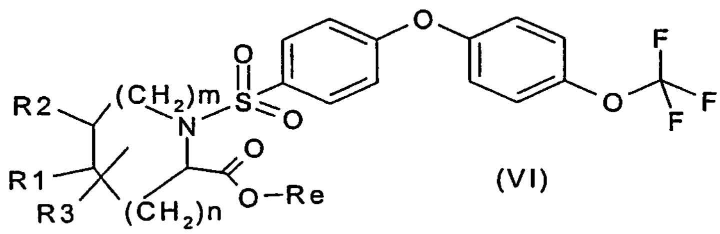

(VI)

worin R1, R2, R3, m und n wie oben definiert sind, und

b) für den Fall Re = Ester eine nach a) hergestellte-Verbindung der Formel VI mit einer Alkalilauge wie NaOH oder LiOH und anschließender Säurebehandlung zu der Verbindung der Formel 1 umsetzt, oder den genannten Ester durch Behandlung mit Mineralsäure wie Salzsäure zur Carbonsäure der Formel VII umsetzt,

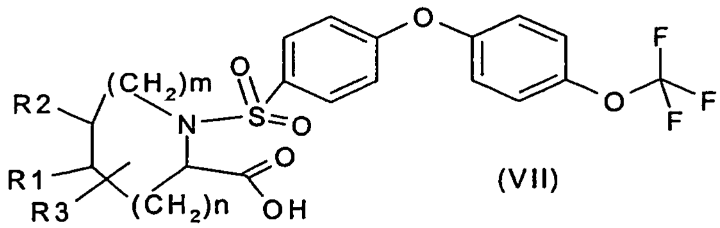

(VII)

und anschließend diese in die Hydroxamsäure, worin X = NH-OH ist, der Formel I umwandelt,

c) eine nach Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder

d) die nach den Verfahren b) oder c) hergestellte Verbindung der Formel I entweder in freier Form isoliert oder im Falle des Vorliegens von sauren oder basischen Gruppen in physiologisch verträgliche Salze umwandelt.

Verbindungen der Art der Formel VI bis VII stellen nur beispielhafte Verbindungen dar, anstatt des Sechsringes können entsprechend der Formel I auch Vierringe, Fünfringe und Siebenringe aufgeführt werden.

Verbindungen des Typs der Formel V lassen sich nach bekannten Vorschriften herstellen.

Beispielsweise können Verbindungen mit n gleich 1 und m gleich 0 (Methanoproline) nach mehreren bekannten Verfahren hergestellt werden. Die Beschreibung einer neueren Synthese findet sich beispielsweise in Tetrahedron 53, 14773-92 (1997).

Beispielsweise können die bicyclischen Grundgerüste der Formel V mit n = 1 und m = 1 nach Formel I durch Hydrierung der Isochinolin-1-carbonsäure oder geeigneter Derivate der Isochinolin-1-carbonsäure, wie der Methyl- oder Ethylester, hergestellt werden. Diese Hydrierung ist beispielsweise in US 5430023, US 5726159 und EP 643073 beschrieben.

Ebenso ist es möglich, zur Herstellung dieser Verbindungen durch Hydrierung 1,2,3,4-Tetrahydroisochinolin-1-carbonsäure und deren Derivate einzusetzen. Dieses Verfahren hat den Vorteil, dass es möglich ist, eine breite Palette an Verfahren zur Synthese der 1,2,3,4-Tetrahydroisochinolin-1-carbonsäuren einzusetzen. Besonders bekannt und breit anwendbar sind beispielsweise Pictet-Spengler-artige Cyclisierungen, wie in US 4902695 beschrieben. Über derartige Verfahren können beispielsweise - je nach Art der eingesetzten Ausgangsprodukte - substituierte Verbindungen erhalten werden, d. h. Verbindungen, in denen die Substituenten R1, R2 und R3 nicht H-Atome bedeuten. Ein neues Beispiel für ringsubstituierte Verbindungen findet sich in WO 2003041641.

Weitere Methoden zur Herstellung der cyclischen Grundgerüste sind beispielsweise über radikalische Cyclisierungsreaktionen möglich und in Tetrahedron 48, 4659-76 (1992) beschrieben.

Andere Verfahren können zur Synthese von Verbindungen des Typs V eingesetzt werden, wenn n = 1 und m = 0 ist. Beispielsweise finden sich beschriebene Synthesen in Tetrahedron 55, 8025 (1999), Tetrahedron Lett. 24, 5339 (1983) bzw. in den Offenlegungsschriften DE 3322530 und DE 3211676. Unter bestimmten Bedingungen kann es sinnvoll sein, Verbindungen des Typs V in N-geschütztem Zustand einzusetzen. Beispielsweise können derart geschützte Verbindungen besser aufgereinigt werden als die freien Iminosäuren, ebenso können diese u. U. auch besser zur Herstellung der enantiomeren- oder diastereomerenreinen Verbindungen eingesetzt werden. Als Schutzgruppen der Aminogruppe können die in "Protective Groups in Organic Synthesis", T.H. Greene, P. G. M. Wuts, Wiley-Interscience, 1999, beschriebenen Gruppen eingesetzt werden. Bevorzugte Amino- oder Imino-Schutzgruppen sind beispielsweise Z, Boc, Fmoc, Aloc, Acetyl, Trifluoracetyl, Benzoyl, Benzyl und ähnliche.

Die Umsetzungen erfolgen beispielsweise wie in WO97/18194 dargestellt. Die Umsetzung gemäß Verfahrensschritt a) erfolgt in Gegenwart einer Base wie KOH, NaOH, LiOH, N-Methyl-morpholin (NMM), N-Ethylmorpholin (NEM), Triethylamin (TEA), Diisopropylethylamin (DIPEA), Pyridin, Collidin, Imidazol oder Natriumcarbonat, in Lösungsmitteln wie Tetrahydrofuran (THF), Dimethylformamid (DMF), Dimethylacetamid, Dioxan, Acetonitril, Toluol, Chloroform oder Methylenchlorid, oder auch in Gegenwart von Wasser. Für den Fall, dass die Umsetzung unter Verwendung von Silylierungsmitteln durchgeführt wird, setzt man beispielsweise N,O-Bis-(trimethylsilyl)acetamid (BSA) oder N,O-Bis-(trimethylsilyl)trifluoracetamid (BSTFA) zur Silylierung der Iminosäure ein, um anschließend die Sulfonamidbildung durchzuführen.

Modifikationen in der Seitenkette F bedeutet, dass beispielsweise eine Nitrogruppe mit dem Metallkatalysator Pd/C hydriert oder mit $SnCl_2$ bzw. Zn unter Standardbedingungen umgesetzt wird und die erhaltene Aminogruppe

anschließend weiter modifiziert werden kann, beispielsweise durch Umsetzung mit Carbonsäurechloriden, Sulfonsäurechloriden, Chlorameisensäureestern, Isocyanaten, Isothiocyanaten oder anderen reaktiven oder aktivierbaren Reagenzien, um zu den Vorstufen der erfindungsgemäßen Verbindungen der Formel I zu gelangen. Für diesen Fall ist es oft günstig, dass Re in Verbindung III ein Ester ist, da im Falle der ungeschützten Carbonsäure mit Nebenreaktionen zu rechnen ist.

Im Verfahrensschritt c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenrreine Ausgangsprodukte einzusetzen. Dadurch können ggf. auch andere oder vereinfachte Verfahren zur Aufreinigung der Endprodukte eingesetzt werden. Diese Ausgangsprodukte wurden zuvor nach Literatur-bekannten Verfahren enantiomeren-oder diastereomerenrein hergestellt. Beispielsweise kann in dem Verfahren zur Herstellung der Decahydroisochinolin-1-carbonsäure entweder direkt die Isochinolin-1-carbonsäure eingesetzt werden, wie oben angeführt und zitiert. Dadurch, dass 3 Stereozentren vorhanden sind, können in diesem Fall maximal 8 Stereoisomere (4 enantiomere Diasteromerenpaare) gebildet werden. Bedingt durch die Art der Herstellung, beispielsweise Hydrierung, sind jedoch bestimmte Stereoisomere stark bevorzugt. So sollte es möglich sein, wie in der Literatur beschrieben, durch geeignete Wahl der Hydrierungsbedingungen (Katalysator, Druck, Lösemittel, Temperatur) z. B. eine starke Bevorzugung der Wasserstoff-Anlagerung an den Positionen der Ringverknüpfung zu erzielen. So kann unter den angegebenen Bedingungen die Bildung der cis-verknüpften Ringe erreicht werden. Somit bliebe dann noch die Position der Carbonsäure zu bestimmen, die Anzahl der möglichen Stereoisomeren wäre bereits auf 4 eingeschränkt. Bedingt durch die Natur des Hydriermechanismus kann besonders leicht eine Anlagerung der Wasserstoffe auf der gleichen Seite wie die der Brückenkopf-Wasserstoffe erfolgen, d. h. hiermit ist eine weitere Einschränkung der Möglichkeit der Isomerenbildung zu erwarten. Somit könnte im günstigsten Fall von der Bildung nur eines Enantiomerenpaares ausgegangen werden. Dieses sollte anschließend durch die oben genannten Methoden in die Enantiomeren auftrennbar sein. Allerdings muss bei diesen Überlegungen auch davon ausgegangen werden, dass niemals eine völlige Stereoselektion stattfindet, sondern dass praktisch immer auch zu mehr oder minder großen Anteilen die anderen Isomeren entstehen bzw. durch geeignete Methoden auch in kleinsten Mengen nachgewiesen werden können. Für den Fall, dass enantiomerenreine 1,2,3,4-Tetrahydroisochinolin-1-carbonsäureDerivate eingesetzt werden, wäre zu erwarten, dass bei gleichen oder ähnlichen Reaktionsbedingungen wie bei der Hydrierung der Isochinolin-1-carbonsäure analoge Überlegungen Gültigkeit besitzen und zu großen Anteilen wieder nur bevorzugte Stereoisomere gebildet werden; in genannten Fall sollte eine starke Bevorzugung eines einzigen Enantiomeren stattfinden, da bei dem Hydrierprozess unter analogen Bedingungen, die zur cis-Ringverknüpfung bei der Hydrierung der Isochinolin-1-carbonsäure führen, hier ebenso wieder nur Anlagerung der H-Atome von einer Seite erfolgen kann und somit analoge Produkte gebildet werden. Durch geeignete 2D-NMR-Experimente, X-Ray-Verfahren wie etwa der Kokristallisation oder andere, sowie Vergleichsanalytik oder chemische Derivatisierung und geeignete Analytik oder chemische Derivatisierung , die zu bekannten und beschriebenen Isomeren führt, kann die Identität der Strukturen festgestellt werden.

Eine andere Möglichkeit zur Synthese enantiomeren- oder diastereomerenreiner Verbindungen besteht darin, geeignet chiral substituierte Ausgangsstoffe einzusetzen, um durch den chiralen Substituenten eine Induktion von Chiralität an anderen Chiralitätszentren zu erreichen. Beispielsweise könnten chirale Glyoxylsäureester in Pictet-Spengler-Cyclisierungen eingesetzt werden, um chirale Tic-Derivate zu erhalten und diese dann, wie oben bereits erwähnt, zu hydrieren.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze, oder Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschfießlich deren stereoisomeren Formen, gemäß Verfahrensschritt d) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali-oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch neue Zwischenprodukte der Formel III,

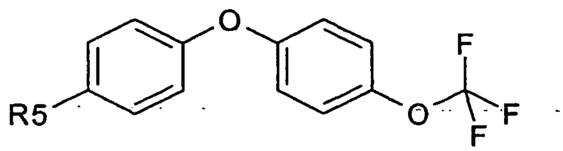
(III)

worin R5 für Wasserstoffatom, $NH_2$, Li, Mg, SH, $S-CH_3$, Cl, Br, I, $Si-(CH_3)_3$, $SO_2-Cl$, $SO_2-Br$, $SO_2-Y$, worin Y einen leicht abspaltbaren Rest darstellt wie einen Aktivester $O-R_y$, wobei Ry für ortho- oder para-Nitrophenyl, 2,4-Dinitrophenyl, oder Pentafluorphenyl steht, oder Y einen Heterocyclus darstellt wie Imidazol, Benzimidazol oder Benzotriazol, wobei die Bindung über den Stickstoff des Heterocyclus erfolgt.

Verfahren zur Aktivester werden beispielsweise in J. Org. Chem. 45, 547 (1980), Indian J. Chem. 25, 1273 (1986), J. Org. Chem. 57, 190 (1992), Nippon Kagaku Kaishi 4, 631-6 (1976) und Tetrahedron Lett. 28, 2115 (1987) beschrieben.

Eine bevorzugte Variante zur Herstellung der Verbindungen der Formel III, worin R5 für $SO_2-Cl$, $SO_2-Br$ oder $SO_3H$ steht, geht vom entsprechend substituierten Diarylether aus. Die Herstellung dieser Aryl-Sulfonsäurechloride und -Sulfonsäuren ist literaturbekannt und kann nach verschiedenen Verfahren erfolgen. Eine häufig angewendete Synthese geht von den Verbindungen der Formel VIII aus,

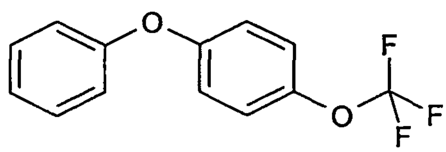
(VIII)

die durch Umsetzung mit Chlorsulfonsäure in die Aryl-Sulfonsäure oder, bei Verwendung eines Überschusses Chlorsulfonsäure, auch direkt in die Aryl-Sulfonylchloride umgewandelt werden können. Die Stellung des einzuführenden Restes ist dabei vom dirigierenden Einfluss weiterer Substituenten abhängig. Phenoxy-Substituenten, wie im vorliegenden Fall, dirigieren eintretende Substituenten wie den Sulfonsäure-Rest in die gewünschte para-Position. Allerdings ist auf die Einhaltung der Reaktionsbedingungen zu achten, da unter Umständen Mehrfachsulfonierungen oder andere unerwünschte Nebenreaktionen auftreten können.

Stellt man zunächst die Sulfonsäure nach dem genannten Verfahren her, ist die Überführung in das Sulfonsäurechlorid nach vielen verschiedenen Methoden möglich. Erfolgreich eingesetzt werden Oxalylchlorid, Phosphoroxychlorid, Phosphorpentachlorid, Thionylchlorid und auch andere Methoden zur Chlorierung. Methoden zur Synthese über Chlorsulfonsäure sind vielfach beschrieben, beispielsweise in Org. Synth. I, 8 und 85 (1941). Zur Einführung des Sulfonsäurerestes in die Verbindung der Formel VIII können weitere bekannte Methoden angewendet werden. Beispielsweise werden eingesetzt:

konzentrierte Schwefelsäure (Recl. Trav. Chim. Pays-Bas 107, 418 (1988), silylierte Schwefelsäure (Bull. Soc. Chim. Fr. 1980, S. 195), J. Am. Chem. Soc. 71, 1593 (1949)), Schwefeltrioxid ((Recl. Trav. Chim. Pays-Bas 111, 215 (1992), Gemische aus Schwefeltrioxid und Schwefeldioxid (J. Prakt. Chem. 22, 290 (1963)), Gemische aus Schwefeltrioxid und konzentrierter Schwefelsäure (J. Prakt. Chem. 93, 183 (1916)).

Eine andere häufig genutzte Methode geht von Arylaminen aus. Diese werden in einer Diazotierungsreaktion zunächst in die Diazoverbindung überführt, beispielsweise durch Umsetzung mit Natriumnitrit in konzentrierter wässriger Salzsäure, und anschließend unter Kupfer-Katalyse, beispielsweise mit CuCl oder CuCl2, in die Sulfonylchloride mit SO2, bevorzugt in Essigsäure, überführt. Siehe zum Beispiel: Bioorg. Med. Chem. Lett. 9, 1251 (1999), J. Med. Chem. 27, 1740 (1984), Org. Synth. 60, 121 (1981), Chem. Ber. 90, 841 (1957), Org. Synth. VII, 508 (1990).

Eine andere Methode geht von Verbindungen der Formel IX aus,

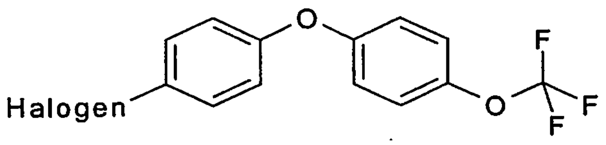

(IX)

worin Halogen für Cl, Br oder I steht. Diese werden mit Alkyllithium, beispielsweise n-BuLi, (Bu steht für Butyl) in die lithiierten Aryle überführt. Diese werden anschließend durch Umsetzung mit $SO_3$-Amin-Adduk (wie Trimethylamin) in die Sulfonsäure überführt. Auch ist die Umsetzung mit $SO_2$ und NCS oder $SO_2$ und $SOCl_2$ beschrieben, wodurch direkt die chlorierten Derivate erhalten werden. Diese Reaktionen sind beispielsweise in J. Org. Chem. 61, 1530 (1996), J. Chem. Soc., Perkin I, 13, 1583 (1996) und Synthesis 1986, S. 852, beschrieben.

Grignard-ähnliche Reaktionen sind ebenfalls beschrieben: Chem. Ber. 128, 575 (1995).

Durch Oxidation von Arylthiolen mit nachfolgender Chlorierung lassen sich ebenfalls Sulfonsäurechloride darstellen: Chem. Lett. 8, 1483 (1992).

Silylierte Phenoxyphenyle lassen sich mit silylierter Chlorsulfonsäure unter Phasen-Transfer-Bedingungen in die Sulfonsäuren überführen (Synthesis 11, 1593 (1998).

Die Herstellung von Verbindungen der Formel III, worin R5 für $SO_3$ steht, lässt sich insbesondere nach zwei unterschiedlichen Verfahren durchführen.

Bevorzugtes Verfahren ist hierbei die Diarylether-Synthese, in der ein Baustein eingesetzt wird, der bereits die Trifluormethoxy-Gruppe trägt. Dies kann bevorzugt beispielsweise entweder das 4-Trifluormethoxybenzole oder eines seiner verwandten Derivate sein, oder aber die 4 substitutierten 4-Trifluormethoxyphenyle, die ein austauschbares F, Cl, Br, I enthalten. Als Reaktionspartner wird im ersten Fall beispielsweise ein Halogenbenzol oder Phenol für den zweiten Fall eingesetzt. Auch andere austauschbare Substitutenten sind möglich, je nach angewandter Synthese und wie etwa in neueren Synthesen beschrieben.

Diese Ausgangsprodukte können entweder nach bekannten Vorschriften hergestellt werden oder sind käuflich erhältlich. Diarylether-Synthesen sind breit beschrieben, eine neuere Synthese beispielsweise in Org. Lett. 6, 913 (2004). Übersichtartikel geben eine Vielzahl an Methoden an, z. B. in: Tetrahedron 56, 5045 (2000); J. Heterocycl. Chem. 36, 1453 (99); Org. Lett. 5, 3799 (2003); Synlett 11, 1734 (2003); Organic Chemistry 2002, S. 1-8; J. Am. Chem. Soc. 119, 10539 (1997).

Ebenso ist es auch möglich, ein geeignet 4-substituiertes Benzolsulfonsäurederivat einzusetzen, wie beispielsweise das 4-Brombenzolsulfonsäurechlorid. Dieses wird mit mindestens 2 Äquivalenten 4-Trifluormethoxyphenol unter den beschriebenen Bedingungen der Arylethersynthese umgesetzt und liefert den korrespondierenden Sulfonsäurearylester des Diarylethers. Anschließend muss noch eine bevorzugt basische Spaltung des Sulfonsäureesters zur Sulfonsäure erfolgen, bevor das Säurechlorid der Formel IV durch Chlorierung erhalten wird.

Als ein weiteres anwendbares Verfahren kann der Aufbau der Trifluormethoxy-Seitenkette aus dem entsprechenden 4-Phenoxyphenol angesehen werden. Allerdings sind - bedingt durch die besonderen Eigenschaften der Trifluormethoxy-Gruppe - nur wenige spezielle Verfahren bekannt, da eine Analogie zu einfachen Alkyl-Ethern nur bedingt gegeben ist. 4-Phenoxyphenol kann mit verschiedenen starken Basen deprotoniert werden. Anschließend wird eine nucleophile Substitutionsreaktion mit Dibromdifluormethan durchgeführt. Das erhaltene Bromdifluormethoxyphenoxyphenol kann mit milden Fluorierungsmethoden anschließend fluoriert werden, beispielsweise mit Pyridin-HF (US4782094 und EP0257415). Die weiteren Umsetzungen zur Verbindung der Formel III können wie oben beschrieben durchgeführt werden.

Die Verbindungen der Formel III können zur Synthese von pharmakologisch aktiven Verbindungen eingesetzt werden. Diese besitzen oftmals eine ähnliche Aktivität wie analoge nicht-fluorierte Derivate. Allerdings sind viele verschiedene Eigenschaften einer Verbindung im Arzneimittelfindungsprozess abzustimmen und zu optimieren. Neben der biologischen Aktivität sind Aufnahme, Verteilung, Metabolismus und Ausscheidung von ganz entscheidender Bedeutung,

so dass eine frühzeitige Testung auf diese Eigenschaften im Arzneifindungsprozess von großer Bedeutung ist und negative Eigenschaften hier zu einer frühzeitigen Beendigung der Profilierung aktiver Substanzen führen kann.

Überraschenderweise wurde nun gefunden, dass Verbindungen, die als Teilstruktur die Verbindung der Formel IV aufweisen, deutlich bessere pharmakokinetische Eigenschaften besitzen als strukturell ähnliche Verbindungen, die unsubstituierte Alkylether oder Alkylfluorid- als Seitenketten aufweisen.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur selektiven Prophylaxe und Therapie all solcher Erkrankungen, an deren Verlauf eine verstärkte Aktivität der Metalloproteinasen beteiligt sind. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie, Herzversagen und septischem Schock. Ebenso eignen sich die Verbindungen zur Prophylaxe von Myocard- und Cerebral-Infarkten.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) und $^1$H-NMR (400 MHz, in DMSO-D6)bestimmt, angegeben sind jeweils der Hauptpeak oder die beiden Hauptpeaks. Temperaturangaben in Grad Celsius, RT bedeutet Raumtemperatur (21 °C bis 24 °C). Verwendete Abkürzungen sind entweder erläutert oder entsprechen den üblichen Konventionen.

Nachfolgend ist die Erfindung an Hand von Beispielen näher erläutert.

Die allgemeinen Vorschriften sind zur Synthese der Verbindungen der Formel I anwendbar.

Allgemeine Vorschrift 1: Sulfonamid aus Sulfonsäurechlorid und Carbonsäure

Die Carbonsäure (6,45 mmol) wurde in 20 ml Dimethylformamid (DMF) gelöst und bei 0°C mit 3 Äquivalenten einer 3N NaOH-Lösung (6,45 ml) versetzt. Nach 10 min tropfte man eine Lösung des Arylsulfonylchlorids (1,1 Äquivalente, 7,1 mmol) in 10 bis 15 ml DMF langsam zu, nach dem Erreichen der Raumtemperatur (RT) wird der Ansatz noch für maximal 12 Stunden (h) bei Temperaturen zwischen 20 °C und 80 °C gerührt. Die genaue Zeit ergibt sich je nach

erfolgtem Umsatz, der massenspektroskopisch festgestellt wurde. Danach wurde das Lösungsmittel unter verminderten Druck entfernt. Anschließend erfolgte wässrige Aufarbeitung (Ausschütteln mit 1 N HCl und gesättigter NaCl-Lösung, Trocknen der organischen Phase wie Essigester, Methylenchlorid oder Chloroform mit Magnesium- oder Natriumsulfat, danach Einengen). Das Rohprodukt wurde entweder direkt weiter umgesetzt oder chromatographisch gereinigt.

Allgemeine Vorschrift 2: Sulfonamid aus Sulfonsäurechlorid und Carbonsäure

Die Carbonsäure wurde in 0,5-2 molarer NaOH gelöst, eventuell unter Zugabe von 10-50 % Tetrahydrofuran (THF) oder DMF. Säurechlorid (1-1,2 Äquivalente, bevorzugt 1,1) wurde in THF gelöst (Konzentration 0,05 bis 1 M) und langsam zugetropft. Am Autotitrator erfolgte automatisch Zugabe von 2 N NaOH bei RT zur pH-Konstanthaltung. Eingestellter pH-Wert: 8 bis 12, bevorzugt 9 bis 11. Nach Beendigung der Reaktion, erkennbar an keinem weiteren NaOH-Verbrauch, wurde das organische Co-Lösungsmittel am Rotationsverdampfer entfernt, die wässrige Lösung oder Suspension mit Essigester versetzt und mit 1 N HCl angesäuert. Nach Abtrennung der organischen Phase und erneuter Extraktion der wässrigen Phase mit Essigester wurden die organischen Phasen vereinigt, über Natriumsulfat getrocknet und anschließend das Lösemittel unter verminderten Druck entfernt. Das Rohprodukt wurde entweder direkt weiter umgesetzt oder chromatographisch gereinigt.

Allgemeine Vorschrift 3: Sulfonamid aus Sulfonsäurechlorid und Carbonsäure.

8 mmol der Iminosäure wurden in 30 ml Acetonitril gelöst oder suspendiert. Bei RT und unter Inertgas ($N_2$) wurden 2,3 g (9 mmol) BSTFA (Bis-(trimethylsilyl)-trifluoracetamid) zugegeben und die Mischung für 2 h unter Rückfluss erhitzt. Zu dieser Lösung gab man 2,84 g (9 mmol) des Sulfonsäurechlorids, gelöst in 30 ml Acetonitril und erhitzte unter Rückflussbedingungen erneut für 3 h. Nach Abkühlen der Reaktionsmischung gab man wässrige 1 N HCl zu, rührte für 1 h, entfernte das Lösemittel unter verminderten Druck am Rotationsverdampfer und gab anschließend Essigsäureethylester oder Chloroform hinzu, trennte die organische Phase ab, extrahierte diese mit gesättigter NaCl-Lösung , trocknete über Natriumsulfat und engte unter verminderten Druck ein. Je nach Reinheit des Reaktionsproduktes konnte dieses direkt weiter umgesetzt werden oder musste vorher über Kieselgel chromatographiert werden.

Allgemeine Vorschrift 4: Herstellung der Hydroxamsäure aus Carbonsäure über Chloroformat-Aktivierung

Die sulfonierte Carbonsäure wurde in 10 ml DMF gelöst und bei 0 °C mit 1,1 Äquivalenten Ethylchloroformiat, 2,2 Äquivalenten N-Ethylmorpholin sowie - nach einer Voraktivierungszeit von 30 min bis 1 h - mit 3 Äquivalenten Trimethylsilylhydroxylamin versetzt. Nachdem man für mindestens 4 h auf 80 °C erhitzte hat, entfernte man das Lösungsmittel unter verminderten Druck und reinigte das Rohprodukt mit chromatographischen Methoden.

Allgemeine Vorschrift 5: Herstellung der Hydroxamsäure durch über das korrespondierende Carbonsäurechlorid

Die sulfonierte Carbonsäure wurde in trockenem Chloroform (Ethanol-frei) vorgelegt (etwa 5 ml für 0,5 mmol) und bei RT mit 3 Äquivalenten Oxalylchlorid versetzt. Anschließend wurde für etwa 30 min auf 45 °C erwärmt. Zur Kontrolle der Chloridbildung wurde eine kleine Probe aus dem Reaktionskolben herausgenommen und mit wenig Benzylamin in THF versetzt. Die vollständige Umsetzung konnte an der quantitativen Benzylamid-Bildung erkannt werden, die Carbonsäure war nicht mehr nachweisbar (Kontrolle durch HPLC-MS). Gegebenenfalls muss für längere Zeit erwärmt werden oder unter Rückflussbedingungen erhitzt werden. Anschließend wurde das Lösemittel unter verminderten Druck abdestilliert, der Rückstand wurde mehrfach in trockenem Toluol aufgenommen und erneut einrotiert. Das Säurechlorid wurde nun erneut in Chloroform (10 ml pro 0,5 mmol) aufgenommen und bei RT mit 3 Äquivalenten O-Trimethylsilylhydroxylamin versetzt. Nach einer Reaktionszeit von mindestens 30 min (Reaktionskontrolle per HPLC-MS) wurde die Reaktionsmischung unter verminderten Druck eingedampft und der Rückstand direkt chromatographisch gereinigt.

Beispiel 1: 4-Trifluormethoxyphenoxy-benzol

4-Trifluormethoxy-brombenzol (10 g, 41,5 mmol), Phenol (3,9 g, 41,5 mmol), Kaliumcarbonat (8,03 g, 58 mmol) und Kupfer-1-Chlorid (103 mg, 1,04 mmol) wurden in trockenem DMF zusammengegeben. Unter Argon wurde für 28 h bei 150 °C gerührt. Anschließend wurde die Reaktionsmischung am Rotationsverdampfer eingeengt, der Rückstand in Essigsäureethylester aufgenommen und mit 10 %iger Natriumcarbonat-Lösung sowie festem Natriumthiosulfat versetzt. Zur Entfernung feiner fester Bestandteile wurden beide Phasen über eine Fritte mit Kieselgur gegeben, anschließend getrennt und die wässrige Phase noch zweimal mit Essigester extrahiert. Die vereinigten organischen Phasen wurden über Natriumsulfat getrocknet und unter verminderten Druck eingedampft. Zur Entfernung von Eduktkomponenten und Nebenprodukten wurde eine Flash-Chromatographie an Kieselgel (Eluens: n-Heptan - Essigsäureethylester

10:1) durchgeführt. Produktfraktionen wurden vereinigt. Ausbeute: 2,5 g, 24 % der Theorie; $^1$H-NMR: 7,09 (m, 4 H); 7,20 (m, 1 H); 7,42 (m, 4 H).
MS: 255.2 (ES+)

Beispiel 2: 4-(4-Trifluormethoxyphenoxy)-benzolsulfonylchlorid

Das Produkt aus Beispiel 1 (2,4 g, 9,44 mmol) wurde in 25 ml Dichlormethan gelöst; unter Kühlung mit Eiswasser wurde langsam eine Lösung der Chlorsulfonsäure in 5 ml Dichlormethan (0,84 g, 7,2 mmol) zugetropft und bei RT für 2,5 h gerührt. Man gab weiteres Dichlormethan dazu und schüttelte mit wenig Wasser aus. Ein feiner Feststoff wurde durch Filtration durch Kieselgur abgetrennt. Die organische Phase wurde abgetrennt, über Natriumsulfat getrocknet und nach Abfiltrieren vom Trockenmittel eingedampft. Zur direkten weiteren Umsetzung wurde in 25 ml Dichlormethan gelöst, Oxalylchlorid (0,823 ml, 1,2 g, 9,44 mmol) wurde langsam zugetropft, 0,5 ml DMF zugegeben und für 1 h bei 40 °C gerührt, über Nacht bei 4 °C aufbewahrt und am nächsten Tag nach Reaktionskontrolle per LC-MS und weitere Zugabe von 0,5 ml Oxalylchlorid erneut für 2 h bei 40 °C gerührt. Die Reaktionsmischung wurde auf Eis gegossen und mit Essigester extrahiert. Die organische Phase wurde mit gesättigter NaCl-Lösung gewaschen, anschließend abgetrennt und über Natriumsulfat getrocknet. Nach dem Abfiltrieren vom Trockenmittel wurde mit Toluol versetzt und unter verminderten Druck eingedampft. Ausbeute: 4,56 g (> 100 %, enthält Salze) $^1$H-NMR (in CDCl3): 6.82; 6.91; 7.12; 7.57 (4m,8H)
MS: 352,0 (ES+)
Hydrolyse (Wasser in Acetonitril) liefert reine Sulfonsäure.
$^1$H-NMR (in DMSO-D6): 6.99; 7.13; 7.40; 7.54 (4 m ("d"), 8 H) MS: 333,2 (ES-)

Beispiel 3: 2-[4-(4-Trifluormethoxyphenoxy)-benzolsulfonyl]-1 ,2,3,4-tetrahydroisochinolin-1-(N-hydroxy)-carboxamid

Schritt 1: Sulfonamidbildung

Tetrahydrochinolin-1-carbonsäure (502 mg, 2,84 mmol) wurde in 60 ml Acetonitril gelöst oder suspendiert. Bei RT und unter Inertgas ($N_2$) wurden 1,85 g (9,07) mmol)

BSA (Bis-(trimethylsilyl)-cetamid) zugegeben und die Mischung für 0,5 h unter Rückfluss erhitzt. Zu dieser Lösung gab man 1,0 g (2,84 mmol) der Verbindung aus Beispiel 2, gelöst in 10 ml Acetonitril und erhitzte unter Rückflussbedingungen erneut für 2 h. Nach Abkühlen der Reaktionsmischung gab man wässrige 1 N HCl zu, rührte für 1 h, entfernte das Lösemittel unter verminderten Druck am Rotationsverdampfer und gab anschließend Essigsäurethylester dazu, trennte die organische Phase ab, extrahierte diese mit gesättigter NaCl-Lösung, trocknete über Natriumsulfat und engte unter verminderten Druck ein. Die Reinheit des Produktes wurde per LC-MS kontrolliert und das erhaltene Rohprodukt daraufhin direkt weiter umgesetzt.

Schritt 2: Hydroxamat-Synthese

Die Verbindung aus Schritt 1 wurde in 40 ml Chloroform gelöst. Anschließend wurde Oxalyl-chlorid (1,585 g, 4,99 mmol, 1,093 ml) innerhalb von 20 min zugetropft und die erhaltene Reaktionsmischung für 2 h auf 40-45 °C erwärmt. Anschließend wurde das Lösemittel unter vermindertem Druck abdestilliert und der erhaltene ölige Rückstand mit Toluol zur Entfernung von etwaigen Oxalylchlorid-Resten oder HCl geschleppt und unter verminderten Druck 15 min belassen. Dann wurde wiederum in Chloroform (40 ml) aufgenommen und bei RT mit O-Trimethylsilylhydroxylamin (0,41 g, 3,9 mmol) versetzt. Nach 2 Stunden wurde das Lösemittel unter verminderten Druck entfernt und der Rückstand in einer kleinen Menge einer Mischung aus Acetonitril-Wasser-0,01 % Trifluoressigsäure zur direkten präparativen RP-HPLC gelöst. Produktfraktionen wurden vereinigt, Acetonitril unter verminderten Druck entfernt und die verbliebene wässrige Phase gefriergetrocknet.
Ausbeute: 580 mg (39 % der Theorie). $^1$H-NMR: 2.7, 2.9 (2 m, 2 H); 3.6, 4.0 (2 m, 2 H) 5.21 (s, 1 H); 7.2 (m, 8 H); 7.45, 7.81 (dd, 4 H); 9.0 (s, br, 1 H); 11.1 (s, 1 H)
MS: 508.09 (ES+)

Die Synthese der Verbindungen gemäß den Beispielen 4 bis 7 wurde in analoger Weise wie oben beschrieben durchgeführt.

Beispiel 4: 2-[4-(4-Trifluoromethoxyphenoxy)-benzolsulfonyl]-decahydro-isochinolin-1 -(N-hydroxy)-carboxamid

$^1$H-NMR: 1,1-1,95 (4 m, 12 H); 3,6 (überlappend mit Wasser; 2 m, 2 H); 4,1 (d, 1 H); 7,12; 7,27; 7,48; 7,77 (2 dd, 8 H); 8,9 (s, br., 1 H); 10,9 (s, 1 H) MS (ES+): 515,21

Beispiel 5: 5-[4-(4-Trifluoromethoxy-phenoxy)-benzolsulfonyl]-octahydro-furo[3,2-c]pyridin-4-carbonsäure

$^1$H-NMR: 1,5-2,1 (4 m, 4 H); 2,55 (m, 1 H); 3,25-3,85 (4 m, 4-5 H, überlappend mit Wasser); 4,28 (d, 1 H); 7,1 (d, 2 H); 7,18; 7,27; 7,48; 7,80 (2 dd, 8 H); 12,8 (s, 1 H) MS (ES+): 488,07

Beispiel 6: 5-[4-(4-Trifluoromethoxy-phenoxy)-benzolsulfonyl]-octahydro-furo[3,2-c]pyridin-4-(N-hydroxy)-carboxamid

MS (ES+): 503,10 (RT 1,442 min; YMC J'sphere ODS H80 20x2, 4 p; 30 °C, 0 min 96 % Wasser, 0,05 % TFA, 2,0 min-95 % Acetonitril; 95 % Acetonitril bis 2,4 min; 4 % Acetonitril 2,45 min; 1 ml/min. inj. vol. 0,4 μl)

Beispiel 7: 2-[4-(4-Trifluoromethoxy-phenoxy)-benzolsulfonyl]-decahydrobenzo[c]azepin-1-(N-hydroxy)-carboxamid

$^1$H-NMR: 1,1-2,3 (m, 14 H); 3,6 (überlappend mit Wasser; 2 m, 2 H); 4,4 (d, 1 H); 7,12; 7,25; 7,48; 7,79 (2 dd, 8 H); 8,7 (s, br., 1 H); 10,5 (s, 1 H) MS (ES+): 529,25

Herstellung der Vergleichsverbindungen

Die Synthese der Vergleichsverbindungen der Tabelle 2 wurde in analoger Weise wie oben beschrieben durchgeführt.

Die analogen Sulfonsäurechloride, die die Methoxy- sowie Trifluormethyl-Seitenkette tragen, sind kommerziell erhältlich. Sulfonamidbildung sowie Hydroxamsäurebildung wird wie oben beschrieben analog durchgeführt. Zur Herstellung der Ethoxy-Verbinung wird von 4-Phenoxyphenol ausgegangen. Nach Standardverfahren der Etherbildung, die dem Fachmann bekannt sind, wird zunächst über Triflataktivierung der Ethylether eingeführt, anschließend erfolgt Umsetzung zum Sulfonsäurechlorid analog wie oben beschrieben. Sulfonamidbildung sowie Herstellung der Hydroxamsäure erfolgt ebenso analog wie oben beschrieben.

Beispiel 8: 2-[4-(4-Methoxyphenoxy)-benzolsulfonyl]-1,2,3,4-tetrahydroisochinolin-1 - carbonsäure

$^1$H-NMR: 2,5 - 2,9 (m, 2 H); 3,5 - 3,8 (m, 2 H); 3,8 (s, aufgesp., 3 H); 5,38 (s, 1 H); 6,95-7,8 (mm, 12 H) MS (ES+): 439,11

Beispiel 9: 2-[4-(4-Methoxyphenoxy)-benzolsulfonyl]-1,2,3,4-tetrahydroisochinolin-1 - N-hydroxycarboxamid

$^1$H-NMR: 2,68, 2,90, 3,50, 3,98 (4 m, 4 H); 3,8 (s, 3 H); 5,20 (s, 1 H); 6,90 - 7,25 (m, 10 H); 7,70 (d, 2 H); 11,0 (s, 1 H), MS (ES+): 454,12

Beispiel 10: 2-[4-(4-Trifluorphenoxy)-benzolsulfonyl]-1,2,3,4-tetrahydroisochinolin-1 - carbonsäure

$^1$H-NMR: 2,45 - 2,9 (m, 2 H); 3,6 - 4,1 (2m, 2 H); 5,45 (s, 1 H); 6,1-7,9 (mm, 12 H); 9,0, 11,1 (2 s, 2 H), MS (ES+): 493,06

Pharmakokinetische Messungen - allgemeine Vorschrift

Zur Untersuchung wurden jeweils 14 oder 16 männliche C57/BL-Mäuse mit einem mittleren Gewicht von 20 bis 28 g verwendet und in zwei Gruppen eingeteilt. Die Tiere hatten freien Zugang zu Futter und Wasser. Die Substanzen wurden in PEG400/Wasser 1:1 gelöst und oral in einer Konzentration von etwa 7,5 mg pro kg (entspricht etwa 0,2 g pro Tier) per Schlundsonde verabreicht. Es wurden jeweils 2 neue Verbindungen sowie eine Vergleichsverbindung vorher separat gelöst, gemischt und gleichzeitig verabreicht (n-in-one-Studie). Nach 0,25, 0,5, 1,2, 4, 6, 8 und 24 h wurden Blutproben entnommen und die Substanzkonzentration per HPLC-MS unter standardisierten Bedingungen wie unten beschrieben quantitativ bestimmt. Die Einzelergebnisse wurden zunächst innerhalb der beiden Gruppen zusammengefasst und anschließend hieraus der Mittelwert gebildet. Zur Berechnung der pharmakokinetischen Parameter wird das "noncompartmental model (extravascular input)" herangezogen.
Die Quantifizierung erfolgte per HPLC-MS-MS. Es wurde ein HPLC-System der Firma Agilent (1100) verwendet, gekoppelt mit einem PE-Sciex API 4000 (Triple-Quadrupol Massen-spektrometer). Als Säule wurde eine ProdigyR 5 μ ODS, Flußrate 0,32 ml/ml, Injektionsvolumen 16 μl. Eluens: Acetonitril - 0,002 % Ammoniumformiat, verwendet. Detektion erfolgte im MS/MS-Modus (Multiple Reaction Monitoring) fokussiert auf Q1 und selektiver Massen (Fragment)-Filtration auf Q3.
Die Aufarbeitung der Plasma-Proben zuvor erfolgte wie folgt: Zumischung von 25 μl 60/40 Acetonitril/0,1 % Formiat,

plus 25 µl interner Standard 5 µg/ml in gleichem Solvens, plus 25 µl Leerplasma oder Sampleplasma, plus 200 µl Acetonitril. Es wurde für 5 min gemixt und anschließend zentrifugiert (3 min, 5000 g) und dann 200 µl in die Messgefäße pipettiert.

Pharmakologische Beispiele

Darstellung und Bestimmung der enzymatischen Aktivität der katalytischen Domäne des humanen Stromelysins (MMP-3) und der Neutrophilen-Kollagenase (MMP-8). Die beiden Enzyme -Stromelysin (MMP-3) und Neutrophilen-Kollagenase (MMP-8) wurden dargestellt nach Ye et al. (Biochemistry; 31 (1992) Seiten 11231-11235). Zur Messung der Enzymaktivität oder der Enzyminhibitorwirkung wurden 10 µl Enzymlösung mit 10 µl einer 3 %igen (v/v) gepufferten Dimethylsulfoxid-Lösung, die gegebenenfalls den Enzyminhibitor enthielt, für 15 Minuten inkubiert. Nach Zugabe von 10 µl einer 3 %igen (v/v) wässrigen Dimethylsulfoxid-Lösung, die 1 mmol/l des Substrates enthielt, wurde die Enzymreaktion fluoreszenzspektroskopisch verfolgt (328 nm (ex) / 393 nm(em)).
Die Enzymaktivität wird dargestellt als Extinktionszunahme/Minute. Die in Tabelle 1 aufgeführten $IC_{50}$-Werte wurden als diejenige Inhibitorkonzentrationen ermittelt, die jeweils zu einer 50%igen Inhibierung des Enzyms führte.
Die Pufferlösung enthielt 0,05 % Brij (Sigma, Deisenhofen, Deutschland) sowie 0,1 mol/l Tris/HCl, 0,1 mol/l NaCl, 0,01 mol/l $CaCl_2$ und 0,1 mol/l Piperazin-N,N'-bis[2-ethan-sulfonsäure] (pH=7,5).
Die MMP-3 Enzymlösung enthielt 2,3 µg/ml, die MMP-8 Enzymlösung 0,6 µg/ml einer der nach Ye et al. dargestellten Enzymdomänen. Die Substratlösung enthielt 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Kollagenase -3 (MMP-13).

Dieses Protein wurde als inaktives Pro-Enzym von der Fa. INVITEK, Berlin, erhalten (Katalog Nr. 30 100 803).
Aktivierung des Proenzyms:
2 Volumenanteile Proenzym wurden mit 1 Volumenanteil APMA-Lösung bei 37 °C für 1,5 Stunden inkubiert. Die APMA-Lösung wurde aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wurde durch Zugabe von 1 mmol/L HCl zwischen 7,0 und 7,5 eingestellt. Nach der Aktivierung des Enzyms wurde dieses mit dem Tris/HCl Puffer auf eine Konzentration von 1,67 µg/mL verdünnt.
Zur Messung der Enzymaktivität wurden 10 µL Enzymlösung mit 10 µL einer 3 %igen (v/v) gepufferten Dimethylsulfoxid-Lösung (Reaktion 1) für 15 Minuten inkubiert. Zur Messung der Enzyminhibitoraktivität wurden 10 µL Enzymlösung mit 10 µL einer 3 %igen (v/v) gepufferten Dimethylsulfoxid-Lösung, die den Enzyminhibitor enthielt, inkubiert (Reaktion 2). Sowohl bei Reaktion 1 als auch bei Reaktion 2 wurde nach Zugabe von 10 µL einer 3 %igen (v/v) wässrigen Dimethylsulfoxid-Lösung, die 0,075 mmol/L des Substrates enthielt, die Enzymreaktion fluoreszenzspektroskopisch verfolgt (328 nm (Extinktion) / 393 nm(Emission)).
Die Enzymaktivität wurde dargestellt als Extinktionszunahme/Minute.
Die Inhibitorwirkung wurde als prozentuale Hemmung nach folgender Formel berechnet:

$$\text{\% Hemmung} = 100 - [(\text{Extinktionszunahme/Minute in Reaktion 2}) / (\text{Extinktionszunahme/Minute in Reaktion 1}) \times 100].$$

Der $IC_{50}$, dies ist die Inhibitorkonzentration, die für eine 50 %ige Hemmung der Enzymaktivität erforderliche ist, wurde grafisch durch Auftragen der prozentualen Hemmungen bei verschiedenen Inhibitorkonzentrationen ermittelt.
Die Pufferlösung enthielt 0,05% Brij (Sigma, Deisenhofen, Deutschland) sowie 0,1 mol/L Tris/HCl, 0,1 mol/L NaCl, 0,01 mol/L $CaCl_2$ (pH=7,5). Die Enzymlösung enthielt 1,67 µg/mL der Enzymdomäne. Die Substratlösung enthielt 0,075 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).
Beispielhafte MMP-Inhibitoren, die mit der beschriebenen Seitenkette überraschenderweise besonders günstige Eigenschaften besitzen: variiert wurde lediglich die Alkyl-Seitenkette.

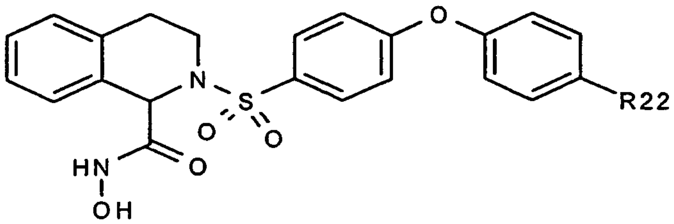

Tabelle 1: Hemmwerte (IC50 in nM) der Verbindungen gegen Matrix-Metalloproteinasen

| Beispiel | R22 | MMP-3 | MMP-8 | MMP-13 |
|---|---|---|---|---|
| 3 | $OCF_3$ | 28 | 3,6 | 2,0 |
| 4 | $OCF_3$ | 69 | 14 | 4 |
| 5 | $OCF_3$ | 380 | 210 | 240 |
| 6 | $OCF_3$ | 24 | 4 | 2 |
| 7 | $OCF_3$ | 24 | 4 | 2 |
| Vergleichsverbindung | | | | |
| 9 | OMe | 23 | 2,3 | 2,2 |

Tabelle 2: Vergleich der PK-Daten der Verbindung mit der erfindungsgemäßen Seitenkette sowie den hierzu ähnlichste Verbindung.

| Beispiel | R22 | Cmax (μg(ml) | t1/2 (h) | AUC (μg/ml*h) |
|---|---|---|---|---|
| 3 | $OCF_3$ | 1,35 | 6,6 | 2,23 |
| Vergleichsverbindungen | | | | |
| | OEt | 0,33 | 4,7 | 0,43 |
| 9 | OMe | 0,45 | 1,5 | 0,25 |
| 10 | $CF_3$ | 0,24 | 1,8 | 0,16 |

Me steht für Methylrest; Et steht für Ethylrest

Cmax ist die maximal erreichte Plasmakonzentration zu einem der Zeitpunkte der Probennahme. AUC "area under the curve", Zeitverlauf der Konzentrationsabfalles und Cmax bestimmen die Größe des Wertes.

Besonders deutlich wird der Unterschied bei Vergleich der besonders relevanten "Area under the curve", AUC. Dieser Wert ist bei der erfindungsgemäßen Trifluormethoxy-Verbindung um etwa den Faktor 5 besser als die Verbindung mit Methoxy-Seitenkette.

**Patentansprüche**

**1.** Verbindung der Formel I

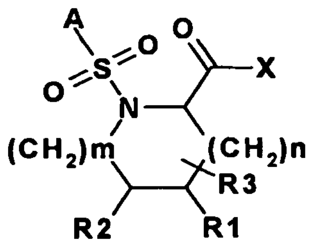

(I)

und/oder alle stereoisomeren Formen der Verbindung der Formel I und/oder Gemische dieser Formen in jedem Verhältnis, und/oder ein physiologisch verträgliches Salz der Verbindung der Formel I, wobei

X für -OH oder -NH-OH steht,
A für einen Rest der Formel II steht,

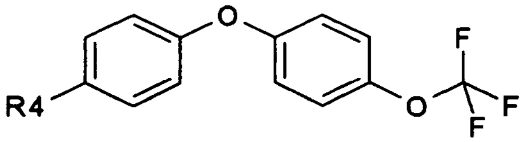

(II)

worin R4 die kovalente Bindung mit dem S-Atom der Formel I bedeutet, R1, R2 und R3 gleich oder verschieden sind und unabhängig voneinander für

1) Wasserstoffatom,
2) -($C_1$-$C_6$)-Alkyl, worin Alkyl unsubstituiert oder ein- oder zweifach durch - ($C_3$-$C_6$)-Cycloalkyl, -($C_2$-$C_4$)-Alkenylen, -($C_2$-$C_6$)-Alkinyl, -($C_6$-$C_{14}$)-Aryl oder Het-Ring substituiert ist,
3) -C(O)-O-R8, worin R8

3)1) Wasserstoffatom,
3)2) -($C_1$-$C_6$)-Alkyl, worin Alkyl unsubstituiert oder ein- oder zweifach durch -($C_3$-$C_6$)-Cycloalkyl, -($C_2$-$C_4$)-Alkenylen, -($C_2$-$C_6$)-Alkinyl, -($C_6$-$C_{14}$)-Aryl, oder Het-Ring oder ein- bis fünffach durch Fluor, substituiert ist,
3)3) -($C_6$-$C_{14}$)-Aryl oder
3)4) Het-Ring bedeutet,

4) -O-R8, worin R8 die oben genannte Bedeutung hat,
5) -($C_3$-$C_6$)-Cycloalkyl,
6) -Halogen,
7) -$NO_2$, oder
8) -CN, stehen, oder
9) R1 und R2 bilden zusammen mit den Kohlenstoffatomen an die sie gebunden sind einen -($C_6$-$C_{14}$)-Aryl-Ring, worin der Ring unsubstituiert oder ein- oder zweifach durch G substituiert ist,
10) R1 und R2 bilden zusammen mit den Kohlenstoffatomen, an die sie gebunden sind, einen 5-, 6- oder 7-gliedrigen Het-Ring, wobei der Ring unsubstituiert oder einfach durch G substituiert ist, oder
11) R1 und R2 bilden zusammen mit den Kohlenstoffatomen, an die sie gebunden sind, ein Indolyl, worin das Indolyl unsubstituiert oder ein- oder zweifach durch G substituiert ist,

G für

1) Wasserstoffatom,
2) Halogen,
3) =O,
4) -($C_1$-$C_6$)-Alkyl, worin Alkyl unsubstituiert oder ein-, zwei- oder dreifach durch Halogen, -($C_3$-$C_6$)-Cycloalkyl, -($C_2$-$C_4$)-Alkenylen, - ($C_2$-$C_6$)-Alkinyl,

-($C_6$-$C_{14}$)-Aryl oder Het-Ring substituiert ist,
5) -($C_6$-$C_{14}$)-Aryl,
6) Het-Ring,
7) -C(O)-O-R10, worin R10

a) -($C_1$-$C_6$)-Alkyl, worin Alkyl unsubstituiert oder ein- oder zweifach durch -($C_3$-$C_6$)-Cycloalkyl, -($C_2$-$C_4$)-Alkenylen, - ($C_2$-$C_6$)-Alkinyl,
-($C_6$-$C_{14}$)-Aryl, oder Het-Ring substituiert ist,
b) -($C_6$-$C_{14}$)-Aryl oder
c) Het-Ring, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl oder Het-Ring substituiert ist,
b) -($C_6$-$C_{14}$)-Aryloder
c) Het-Ring, bedeutet,

10) -C(S)-NH-R11, worin R11 wie oben definiert ist, bedeutet,
11) -O-R12, worin R12

a) Wasserstoffatom,
b) -($C_1$-$C_6$)-Alkyl, worin Alkyl unsubstituiert oder ein-, zwei-oder dreifach durch Halogen, -($C_3$-$C_6$)-Cycloalkyl, -($C_2$-$C_4$)-Alkenylen,
-($C_2$-$C_6$)-Alkinyl, -($C_6$-$C_{14}$)-Aryl oder Het-Ring substituiert ist,
c) -($C_6$-$C_{14}$)-Aryl,
d) Het-Ring,
e) -C(O)-O-R13, worin R13

e)1) -($C_1$-$C_6$)-Alkyl, worin Alkyl unsubstituiert oder ein-oder zweifach durch -($C_3$-$C_6$)-Cycloalkyl, -($C_2$-$C_4$)-Alkenylen,
-($C_2$-$C_6$)-Alkinyl, -($C_6$-$C_{14}$)-Aryl, oder Het-Ring substituiert ist,
e)2) -($C_6$-$C_{14}$)-Aryl oder
e)3) Het-Ring, bedeutet,

f) -C(S)-O-R13, worin R13 wie oben definiert ist,
g) -C(O)-NH-R14, worin R14

g)1) -($C_1$-$C_6$)-Alkyl, worin Alkyl unsubstituiert oder ein-oder zweifach durch -($C_3$-$C_6$)-Cycloalkyl, -($C_2$-$C_4$)-Alkenylen,
-($C_2$-$C_6$)-Alkinyl, -($C_6$-$C_{14}$)-Aryl oder Het-Ring substituiert ist,
g)2) -($C_6$-$C_{14}$)-Aryl oder
g)3) Het-Ring bedeutet, oder

h) -C(S)-NH-R14, worin R14 wie oben definiert ist, bedeutet,

12) -C(O)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN,
16) -N(R15)-R12, worin R15

16)1) Wasserstoffatom,
16)2) -($C_1$-$C_6$)-Alkyl oder
16)3) -$SO_2$-($C_1$-$C_6$)-Alkyl, worin Alkyl unsubstituiert oder ein-oder zweifach durch -($C_3$-$C_6$)-Cycloalkyl, -($C_2$-$C_4$)-Alkenylen,
-($C_2$-$C_6$)-Alkinyl, -($C_6$-$C_{14}$)-Aryl oder Het-Ring substituiert ist, bedeutet und R12 wie oben definiert ist,

oder

17) $-SO_2-N(R12)-R16$, worin R12 wie oben definiert ist und worin R16

17)1) Wasserstoffatom,
17)2) $-(C_1-C_6)$-Alkyl, worin Alkyl unsubstituiert oder ein- oder zweifach durch $-(C_3-C_6)$-Cycloalkyl, $-(C_2-C_4)$-Alkenylen, - $(C_2-C_6)$-Alkinyl,
$-(C_6-C_{14})$-Aryl oder Het-Ring substituiert ist,
17)3) -C(O)-O-R8, worin R8 die oben genannte Bedeutung hat,
17)4) -O-R8, worin R8 die oben genannte Bedeutung hat, oder
17)5) $-(C_3-C_6)$-Cycloalkyl bedeutet, steht, und

m und n gleich oder verschieden sind und für die Zahlen Null, 1, 2 oder 3 stehen, mit der Maßgabe, dass die Summe von m und n den Betrag Null, 1, 2 oder 3 hat.

**2.** Verbindung der Formel I gemäß Anspruch 1, wobei

X für -OH öder-NH-OH steht,
A für einen Rest der Formel II steht,
R1 und R2 bilden zusammen mit den Kohlenstoffatomen an die sie gebunden sind

a) einen $-(C_6-C_{14})$-Aryl-Ring, worin Aryl ein Rest aus der Reihe Phenyl, Naphthyl, 1-Naphthyl, 2-Naphthyl, Anthryl oder Fluorenyl bedeutet und unsubstituiert oder ein- oder zweifach durch G substituiert ist, oder
b) einen 5-, 6- oder 7-gliedrigen Het-Ring, worin Het-Ring ein Rest aus der Reihe Furan, Imidazol, Isoxazol, Oxazol Pyrazin, Pyrazol, Pyridazin, Pyridin, Pyrimidin, Thiazol oder Thiophen bedeutet und unsubstituiert oder ein- oder zweifach durch G substituiert ist, oder
c) ein Indolyl, worin das Indolyl unsubstituiert oder ein- oder zweifach durch G substituiert ist,

G für

1) Wasserstoffatom,
2) Fluor, Chlor, Brom oder Jod,
3) =O,
4) $-(C_1-C_6)$-Alkyl, worin Alkyl unsubstituiert oder ein- zwei- oder dreifach durch Halogen, $-(C_3-C_6)$-Cycloalkyl, $-(C_2-C_6)$-Alkinyl, $-(C_6-C_{14})$-Aryl, worin Aryl für Phenyl oder Naphthyl steht, oder Het-Ring, worin der Het-Ring ein Rest aus der Reihe Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, lsobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathünyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl ist, substituiert ist,
5) $-(C_6-C_{14})$-Aryl, worin Aryl für Phenyl oder Naphthyl steht,
6) Het-Ring, worin Het wie oben definiert ist,
7) -C(O)-O-R10, worin R10

a) $-(C_1-C_6)$-Alkyl, worin Alkyl unsubstituiert oder ein- oder zweifach durch $-(C_3-C_6)$-Cycloalkyl,

-($C_2$-$C_6$)-Alkinyl, -($C_6$-$C_{14}$)-Aryl, oder Het-Ring substituiert ist, worin Aryl für Phenyl oder Naphthyl steht und Het wie oben definiert ist,
b) -($C_6$-$C_{14}$)-Aryl, worin Aryl für Phenyl oder Naphthyl steht, oder
c) Het-Ring, worin Het wie oben definiert ist, bedeutet,

8) -C(S)-O-R10, worin R10 wie oben definiert ist,
9) -C(O)-NH-R11, worin R11

a) -($C_1$-$C_6$)-Alkyl, worin Alkyl unsubstituiert oder ein- oder zweifach durch -($C_3$-$C_6$)-Cycloalkyl, -($C_6$-$C_{14}$)-Aryl oder Het-Ring substituiert ist, worin Aryl für Phenyl oder Naphthyl steht und Het wie oben definiert ist,
b) -($C_6$-$C_{14}$)-Aryl, worin Aryl für Phenyl oder Naphthyl steht, oder
c) Het-Ring, worin Het wie oben definiert ist, bedeutet,

10) -C(S)-NH-R11, worin R11 wie oben definiert ist, bedeutet,
11) -O-R12, worin R12

a) Wasserstoffatom,
b) -($C_1$-$C_6$)-Alkyl, worin Alkyl unsubstituiert oder ein-, zwei-oder dreifach durch Halogen, -($C_3$-$C_6$)-Cycloalkyl, -($C_2$-$C_6$)-Alkinyl,
-($C_6$-$C_{14}$)-Aryl oder Het-Ring substituiert ist, worin Aryl für Phenyl oder Naphthyl steht und Het wie oben definiert ist,
c) -($C_6$-$C_{14}$)-Aryl, worin Aryl für Phenyl oder Naphthyl steht,
d) Het-Ring, worin Het wie oben definiert ist,
e) -C(O)-O-R13, worin R13

e)1) -($C_1$-$C_6$)-Alkyl, worin Alkyl unsubstituiert oder ein-oder zweifach durch -($C_3$-$C_6$)-Cycloalkyl, -($C_2$-$C_6$)-Alkinyl,
-($C_6$-$C_{14}$)-Aryl oder Het-Ring substituiert ist, worin Aryl für Phenyl oder Naphthyl steht und Het wie oben definiert ist,
e)2) -($C_6$-$C_{14}$)-Aryl, worin Aryl für Phenyl oder Naphthyl steht, oder
e)3) Het-Ring, worin Het wie oben definiert ist, bedeutet,

f) -C(S)-O-R13, worin R13 wie oben definiert ist,
g) -C(O)-NH-R14, worin R14

g)1) -($C_1$-$C_6$)-Alkyl, worin Alkyl unsubstituiert oder ein-oder zweifach durch -($C_3$-$C_6$)-Cycloalkyl, -($C_2$-$C_6$)-Alkinyl,
-($C_6$-$C_{14}$)-Aryl oder Het-Ring substituiert ist, worin
Aryl für Phenyl oder Naphthyl steht und Het wie oben definiert ist,
g)2) -($C_6$-$C_{14}$)-Aryl, worin Aryl für Phenyl oder Naphthyl steht, oder
g)3) Het-Ring bedeutet, worin Het wie oben definiert ist,

oder
h) -C(S)-NH-R14, worin R14 wie oben definiert ist, bedeutet,

12) -C(O)-R10, worin R10 wie oben definiert ist,
13) -$S(O)_p$-R12, worin R12 wie oben definiert ist und p die ganzen Zahlen Null, 1 oder 2 bedeutet,
14) -$NO_2$,
15) -CN oder
16) -N(R15)-R12, worin R15

16)1) Wasserstoffatom,
16)2) -($C_1$-$C_6$)-Alkyl oder
16)3) -$SO_2$-($C_1$-$C_6$)-Alkyl, worin Alkyl unsubstituiert oder ein- oder zweifach durch -($C_3$-$C_6$)-Cycloalkyl, -($C_2$-$C_6$)-Alkinyl, -($C_6$-$C_{14}$)-Aryl oder Het-Ring substituiert ist, worin Aryl für Phenyl oder Naphthyl steht und Het wie oben definiert ist, bedeutet und R12 wie oben definiert ist, oder

17) $-SO_2-N(R12)-R16$, worin R12 wie oben definiert ist und worin R16

17)1) Wasserstoffatom,
17)2) $-(C_1-C_6)$-Alkyl, worin Alkyl unsubstituiert oder ein- oder zweifach durch $-(C_3-C_6)$-Cycloalkyl, $-(C_2-C_6)$-Alkinyl, $-(C_6-C_{14})$-Aryl oder Het-Ring substituiert ist, worin Aryl für Phenyl oder Naphthyl steht und Het wie oben definiert ist,
17)3) -C(O)-O-R8, worin R8 die oben genannte Bedeutung hat,
17)4) -O-R8, worin R8 die oben genannte Bedeutung hat, oder
17)5) $-(C_3-C_6)$-Cycloalkyl bedeutet, steht, und

m für die Zahl 1 oder 2 steht und n für die Zahl Null steht,
m für die Zahl 1 steht und n für die Zahl zwei steht oder
m und n gleich sind und jeweils für die Zahl 1 stehen.

**3.** Verbindung der Formel I gemäß der Ansprüche 1 oder 2, wobei

X für -OH oder -NH-OH steht,
A für einen Rest der Formel II steht,
m für die Zahl 1 steht und n für die Zahl zwei steht oder
m und n gleich sind und jeweils für die Zahl 1 stehen, und
R1 und R2 bilden zusammen mit den Kohlenstoffatomen an die sie gebunden sind ein Phenyl oder Tetrahydrofuranyl.

**4.** Verbindung der Formel I gemäß einem oder mehreren der Ansprüche 1 bis 3, **dadurch gekennzeichnet dass** es die Verbindung

2-[4-(4-Trifluormethoxy-phenoxy)-benzolsulfonyl]-1,2, 3,4-tetrahydro-isochinolin-1-carbonsäure-hydroxyamid,
5-[4-(4-Trifluoromethoxy-phenoxy)-benzolsulfonyl]-octahydro-furo[3,2-c]pyridin-4-carbonsäure oder
5-[4-(4-Trifluoromethoxy-phenoxy)-benzolsulfonyl]-octahydro-furo[3,2-c]pyridin-4-(N-hydroxy)-carboxamid ist.

**5.** Verfahren zur Herstellung der Verbindung der Formel I gemäß einem oder mehreren der Ansprüche 1 bis 4, **dadurch gekennzeichnet, dass** man

a) eine Verbindung der Formel III

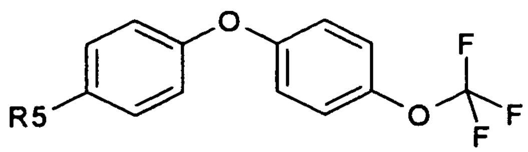

(III)

worin R5 für Wasserstoffatom, $NH_2$, Li, Mg. SH, $S-CH_3$, Cl, Br, I oder $Si-(CH_3)_3$ steht,
in eine Verbindung der Formel IV überführt,

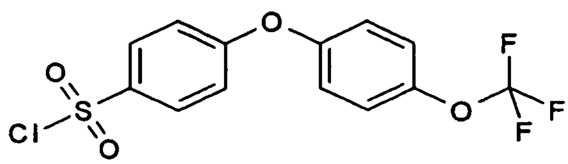

(IV)

und mit einer Verbindung der Formel V,

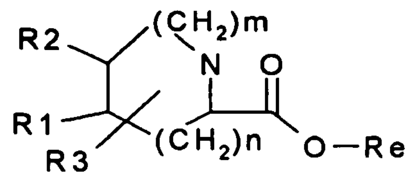

(V)

worin R1, R2, R3, m und n wie in Formel I definiert sind und Re ein Wasserstoffatom oder eine Ester-Schutzgruppe darstellt,
in Gegenwart einer Base oder nach Silylierung mit einem geeigneten Silylierungsmittel zu einer Verbindung der Formel VI umsetzt,

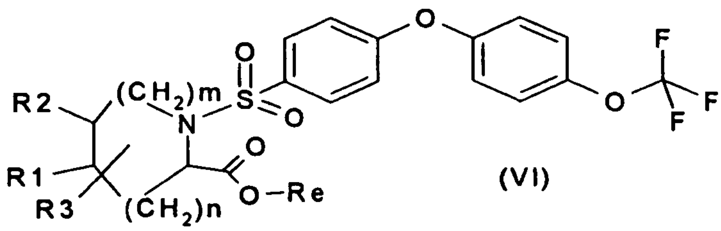

(VI)

worin R1, R2, R3, m und n wie oben definiert sind, und
b) für den Fall Re = Ester eine nach a) hergestellte Verbindung der Formel VI mit einer Alkalilauge wie NaOH oder LiOH und anschließender Säurebehandlung zu der Verbindung der Formel I umsetzt, oder den genannten Ester durch Behandlung mit Mineralsäure wie Salzsäure zur Carbonsäure der Formel VII umsetzt,

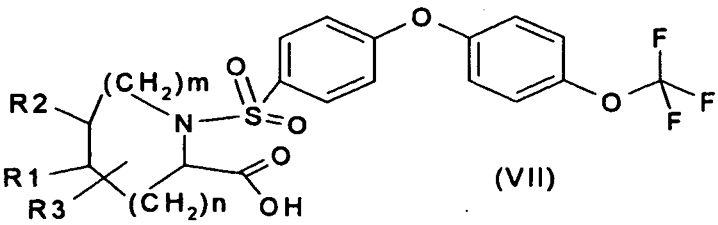

(VII)

und anschließend diese in die Hydroxamsäure, worin X = NH-OH ist, der Formel I umwandelt,
c) eine nach Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren b) oder c) hergestellte Verbindung der Formel 1 entweder in freier Form isoliert oder im Falle des Vorliegens von sauren oder basischen Gruppen in physiologisch verträgliche Salze umwandelt.

**6.** Verbindung der Formel III,

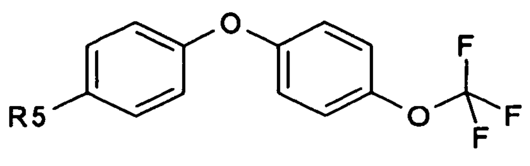

(III)

worin R5 für Wasserstoffatom, Li, Mg, S-$CH_3$, Cl, Br, I, Si-$(CH_3)_3$, $SO_2$-Cl, $SO_2$-Br, $SO_2$-Y, worin Y einen leicht abspaltbaren Rest darstellt wie einen Aktivester O-$R_y$, wobei Ry für ortho- oder para-Nitrophenyl, 2,4-Dinitrophenyl, oder Pentafluorphenyl steht, oder Y einen Heterocyclus darstellt wie Imidazol, Benzimidazol oder Benzotriazol,

wobei die Bindung über den Stickstoff des Heterocyclus erfolgt.

7. Verfahren zur Herstellung der Verbindung der Formel III gemäß Anspruch 6, **dadurch gekennzeichnet dass** man

a) die Verbindung der Formel VIII,

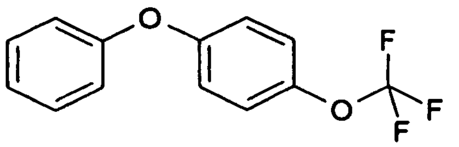

(VIII)

durch Umsetzung mit Chlorsulfonsäure in die entsprechende Aryl-Sulfonsäure oder bei Verwendung eines Überschusses an Chlorsulfonsäure direkt in das Aryl-Sulfonylchlorid überführt, oder
b) die Verbindung der Formel IX,

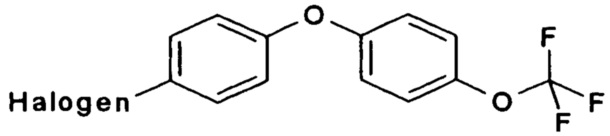

(IX)

worin Halogen für Cl, Br oder I steht, mit Alkyllithium, wie n-BuLi in die lithiierten Aryle überführt und diese gegebenenfalls anschließend durch Umsetzung mit $SO_3$-Amin-Addukten wie Trimethylamin in die entsprechenden Sulfonsäuren überführt.

8. Arzneimittel, **gekennzeichnet durch** einen wirksamen Gehalt an mindestens einer Verbindung der Formel I gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

9. Verwendung der Verbindung der Formel I gemäß einem oder mehreren der Ansprüche 1 bis 4 zur Herstellung eines Arzneimittels zur selektiven Prophylaxe und Therapie all solcher Erkrankungen, an deren Verlauf eine verstärkte Aktivität der Metalloproteinasen beteiligt sind wie degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen oder Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels oder Behandlung der Ulceration, Atherosklerose und Stenosen oder Behandlung von Entzündungen, Krebserkrankungen, Tumormetastasenbildung, Kachexie, Anorexie, Herzversagen und septischem Schock oder Prophylaxe von Myocard-und Cerebral-Infarkten.

## Claims

1. A compound of the formula I

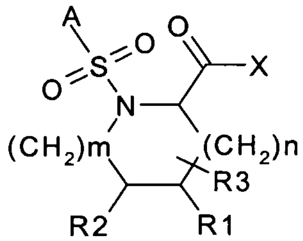

(I)

and/or all stereoisomeric forms of the compound of the formula I and/or mixtures of these forms in any ratio, and/or a physiologically tolerated salt of the compound of the formula I, where

X is -OH or-NH-OH,
A is a radical of the formula II

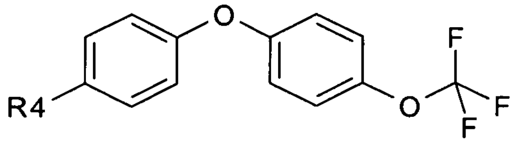

(II)

in which R4 means the covalent bond to the S atom of the formula I,
R1, R2 and R3 are identical or different and are independently of one another

1) hydrogen atom,
2) -($C_1$-$C_6$)-alkyl in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl or Het ring,
3) -C(O)-O-R8 in which R8 is

3)1) hydrogen atom,
3)2) -($C_1$-$C_6$)-alkyl in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl, or Het ring or once to five times by fluorine,
3)3) -($C_6$-$C_{14}$)-aryl or
3)4) Het ring,

4) -O-R8 in which R8 has the abovementioned meaning,
5) -($C_3$-$C_6$)-cycloalkyl,
6) -halogen,
7) -$NO_2$, or
8) -CN, or
9) R1 and R2 form together with the carbon atoms to which they are bonded a -($C_6$-$C_{14}$)-aryl ring in which the ring is unsubstituted or substituted once or twice by G,
10) R1 and R2 form together with the carbon atoms to which they are bonded a 5-, 6- or 7-membered Het ring, where the ring is unsubstituted or substituted once by G, or
11) R1 and R2 form together with the carbon atoms to which they are bonded an indolyl in which the indolyl is unsubstituted or substituted once or twice by G,

G is

1) hydrogen atom,
2) halogen,
3) =O,
4) -($C_1$-$C_6$)-alkyl in which alkyl is unsubstituted or substituted once, twice or three times by halogen, -($C_3$-$C_6$)-cycloalkyl, -($C_2$-$C_4$)-alkenylene, -($C_2$-$C_6$)-alkynyl, -($C_6$-$C_{14}$)-aryl or Het ring,
5) -($C_6$-$C_{14}$)-aryl,
6) Het ring,
7) -C(O)-O-R10, in which R10 is

a) -($C_1$-$C_6$)-alkyl, in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl or Het ring,
b) -($C_6$-$C_{14}$)-aryl or
c) Het ring,

8) -C(S)-O-R10 in which R10 is as defined above,
9) -C(O)-NH-R11 in which R11 is

a) -($C_1$-$C_6$)-alkyl in which alkyl is unsubstituted or substituted once or twice by -($C_3$-$C_6$)-cycloalkyl, -($C_6$-$C_{14}$)-aryl or Het ring,
b) -($C_6$-$C_{14}$)-aryl or
c) Het ring,

10) -C(S)-NH-R11 in which R11 is as defined above,
11) -O-R12 in which R12 is

a) hydrogen atom,
b) -($C_1$-$C_6$)-alkyl in which alkyl is unsubstituted or substituted once, twice or three times by halogen, -($C_3$-$C_6$)-cycloalkyl, -($C_2$-$C_4$)-alkenylene, -($C_2$-$C_6$)-alkynyl, -($C_6$-$C_{14}$)-aryl or Het ring,
c) -($C_6$-$C_{14}$)-aryl,
d) Het ring,
e) -C(O)-O-R13 in which R13 is

e)1) -($C_1$-$C_6$)-alkyl in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl, or Het ring,
e)2) -($C_6$-$C_{14}$)-aryl or
e)3) Het ring,

f) -C(S)-O-R1,3 in which R13 is as defined above,
g) -C(O)-NH-R14 in which R14 is

g)1) -($C_1$-$C_6$)-alkyl in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl or Het ring,
g)2) -($C_6$-$C_{14}$)-aryl or
g)3) Het ring, or

h) -C(S)-NH-R14 in which R14 is as defined above,

12) -C(O)-R10 in which R10 is as defined above,
13) -S(O)p-R12 in which R12 is as defined above, and p is the integers zero, 1 or 2,
14) -$NO_2$,
15) -CN,
16) -N(R15)-R12 in which R15 is

16)1) hydrogen atom,
16)2) -($C_1$-$C_6$)-alkyl or
16)3) -$SO_2$-($C_1$-$C_6$)-alkyl in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl or Het ring, and R12 is as defined above, or

17) -$SO_2$-N(R12)-R16 in which R12 is as defined above, and in which R16 is

17)1) hydrogen atom,
17)2) -($C_1$-$C_6$)-alkyl in which alkyl is unsubstituted or substituted once or twice by -($C_3$-$C_6$)-cycloalkyl, -($C_2$-$C_4$)-alkenylene, -($C_2$-$C_6$)-alkynyl, -($C_6$-$C_{14}$)-aryl or Het ring,
17)3) -C(O)-O-R8 in which R8 has the abovementioned meaning,
17)4) -O-R8 in which R8 has the abovementioned meaning, or
17)5) -($C_3$-$C_6$)-cycloalkyl, and

m and n are identical or different and are the numbers zero, 1, 2 or 3, with the proviso that the total of m and n amounts to zero, 1, 2 or 3.

**2.** A compound of the formula I as claimed in claim 1, where

X is -OH or-NH-OH,
A is a radical of the formula II,

R1 and R2 form together with the carbon atoms to which they are bonded

a) a -($C_6$-$C_{14}$)-aryl ring in which aryl is a radical from the series phenyl, naphthyl, 1-naphthyl, 2-naphthyl, anthryl or fluorenyl, and is unsubstituted or substituted once or twice by G, or
b) a 5-, 6- or 7-membered Het ring in which Het ring is a radical from the series furan, imidazole, isoxazole, oxazole, pyrazine, pyrazole, pyridazine, pyridine, pyrimidine, thiazole or thiophene, and is unsubstituted or substituted once or twice by G, or
c) an indolyl in which the indolyl is unsubstituted or substituted once or twice by G,

G is

1) hydrogen atom,
2) fluorine, chlorine, bromine or iodine,
3) =O,
4) -($C_1$-$C_6$)-alkyl in which alkyl is unsubstituted or substituted once, twice or three times by halogen, -($C_3$-$C_6$)-cycloalkyl, -($C_2$-$C_6$)-alkynyl, -($C_6$-$C_{14}$)-aryl, in which aryl is phenyl or naphthyl, or Het ring in which the Het ring is a radical from the series acridinyl, azepinyl, azetidinyl, aziridinyl, benzimidazalinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, deca-hydroquinolinyl, dibenzofuranyl, dibenzothiophenyl, dihydrofuran[2,3-b]-tetrahydrofuranyl, dihydrofuranyl, dioxolyl, dioxanyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purynyl, pyranyl, pyrazinyl, pyroazolidinyl, pyrazolinyl, pyrazolyl, pyridazinyl, pryidooxazolyl, pyridoimidazolyl, pyridothiazolyl, pyridothiophenyl, pyridinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiomorpholinyl, thiophenyl, triazinyl, 1,2,3-triazolyl, 1,2,4-triazolyl, 1,2,5-triazolyl, 1,3,4-triazolyl and xanthenyl,
5) -($C_6$-$C_{14}$)-aryl in which aryl is phenyl or naphthyl,
6) Het ring in which Het is as defined above,
7) -C(O)-O-R10 in which R10 is

a) -($C_1$-$C_6$)-alkyl in which alkyl is unsubstituted or substituted once or twice by -($C_3$-$C_6$)-cycloalkyl, -($C_2$-$C_6$)-alkynyl, -($C_6$-$C_{14}$)-aryl, or Het ring, in which aryl is phenyl or naphthyl, and Het is as defined above,
b) -($C_6$-$C_{14}$)-aryl in which aryl is phenyl or naphthyl, or
c) Het ring in which Het is as defined above,

8) -C(S)-O-R10 in which R10 is as defined above,
9) -C(O)-NH-R11 in which R11 is

a) -($C_1$-$C_6$)-alkyl in which alkyl is unsubstituted or substituted once or twice by -($C_3$-$C_6$)-cycloalkyl, -($C_6$-$C_{14}$)-aryl or Het ring, in which aryl is phenyl or naphthyl, and Het is as defined above,
b) -($C_6$-$C_{14}$)-aryl in which aryl is phenyl or naphthyl, or
c) Het ring in which Het is as defined above,

10) -C(S)-NH-R11 in which R11 is as defined above,
11) -O-R12 in which R12 is

a) hydrogen atom,
b) -($C_1$-$C_6$)-alkyl in which alkyl is unsubstituted or substituted once, twice or three times by halogen, -($C_3$-$C_6$)-cycloalkyl, -($C_2$-$C_6$)-alkynyl, -($C_6$-$C_{14}$)-aryl or Het ring, in which aryl is phenyl or naphthyl, and Het is as defined above,

c) -($C_6$-$C_{14}$)-aryl in which aryl is phenyl or naphthyl,
d) Het ring in which Het is as defined above,
e) -C(O)-O-R13 in which R13 is

e)1) -($C_1$-$C_6$)-alkyl in which alkyl is unsubstituted or substituted once or twice by -($C_3$-$C_6$)-cycloalkyl, -($C_2$-$C_6$)-alkynyl, -($C_6$-$C_{14}$)-aryl or Het ring, in which aryl is phenyl or naphthyl, and Het is as defined above,
e)2) -($C_6$-$C_{14}$)-aryl in which aryl is phenyl or naphthyl, or
e)3) Het ring in which Het is as defined above,

f) -C(S)-O-R13 in which R13 is as defined above,
g) -C(O)-NH-R14 in which R14 is

g)1) -($C_1$-$C_6$)-alkyl in which alkyl is unsubstituted or substituted once or twice by -($C_3$-$C_6$)-cycloalkyl, -($C_2$-$C_6$)-alkynyl, -($C_6$-$C_{14}$)-aryl or Het ring, in which aryl is phenyl or naphthyl, and Het is as defined above,
g)2) -($C_6$-$C_{14}$)-aryl in which aryl is phenyl or naphthyl, or
g)3) Het ring in which Het is as defined above,

or
h) -C(S)-NH-R14 in which R14 is as defined above,

12) -C(O)-R10 in which R10 is as defined above,
13) -$S(O)_p$-R12 in which R12 is as defined above, and p is the integers zero, 1 or 2,
14) -$NO_2$,
15) -CN or
16) -N(R15)-R12 in which R15 is

16)1) hydrogen atom,
16)2) -($C_1$-$C_6$)-alkyl or
16)3) -$SO_2$-($C_1$-$C_6$)-alkyl in which alkyl is unsubstituted or substituted once or twice by -($C_3$-$C_6$)-cycloalkyl, -($C_2$-$C_6$)-alkynyl, -($C_6$-$C_{14}$)-aryl or Het ring, in which aryl is phenyl or naphthyl, and Het is as defined above, and R12 is as defined above, or

17) -$SO_2$-N(R12)-R16 in which R12 is as defined above, and in which R16 is

17)1) hydrogen atom,
17)2) -($C_1$-$C_6$)-alkyl in which alkyl is unsubstituted or substituted once or twice by -($C_3$-$C_6$)-cycloalkyl, -($C_2$-$C_6$)-alkynyl, -($C_6$-$C_{14}$)-aryl or Het ring, in which aryl is phenyl or naphthyl, and Het is as defined above,
17)3) -C(O)-O-R8 in which R8 has the abovementioned meaning,
17)4) -O-R8 in which R8 has the abovementioned meaning, or
17)5) -($C_3$-$C_6$)-cycloalkyl, and

m is the number 1 or 2 and n is the number zero,
m is the number 1 and n is the number two or
m and n are identical and each is the number 1.

**3.** A compound of the formula I as claimed in claims 1 or 2, where

X is -OH or -NH-OH,
A is a radical of the formula II,
m is the number 1 and n is the number two or
m and n are identical and each is the number 1 and
R1 and R2 form together with the carbon atoms to which they are bonded a phenyl or tetrahydrofuranyl.

**4.** A compound of the formula I as claimed in one or more of claims 1 to 3, which is the compound

2-[4-(4-trifluoromethoxyphenoxy)benzenesulfonyl]-1,2,3,4-tetrahydroisoquinoline-1-hyd roxycarboxamide, 5-[4-(4-trifluoromethoxyphenoxy)benzenesulfonyl]-octahydrofuro[3,2-c]pyridine-4-carboxylic acid or 5-[4-(4-trifluoromethoxyphenoxy)benzenesulfonyl]-octahydrofuro[3,2-c]pyridine-4-(N-hydroxy)-carboxamide.

**5.** A process for preparing the compound of the formula I as claimed in one or more of claims 1 to 4, which comprises

a) converting a compound of the formula III

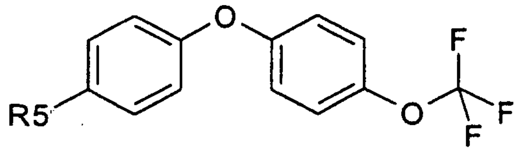

(III)

in which R5 is hydrogen atom, $NH_2$, Li, Mg, SH, S-$CH_3$, Cl, Br, I or Si-$(CH_3)_3$, into a compound of the formula IV,

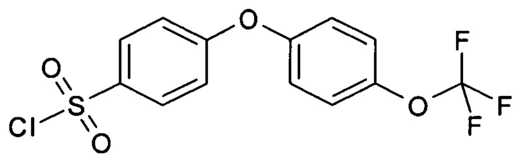

(IV)

and reacting with a compound of the formula V,

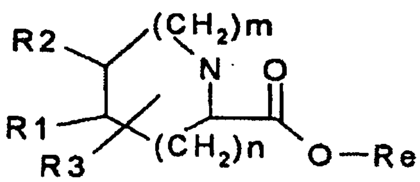

(V)

in which R1, R2, R3, m and n are as defined in formula I, and Re is a hydrogen atom or an ester protective group, in the presence of a base or after silylation with a suitable silylating agent to give a compound of the formula VI,

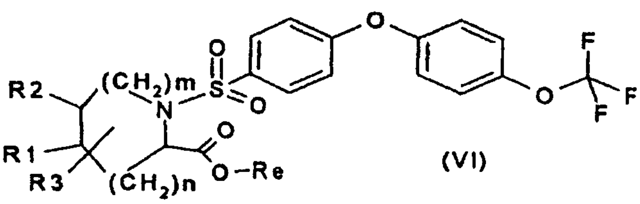

(VI)

in which R1, R2, R3, m and n are as defined above, and

b) in the case where Re = ester reacting a compound of the formula VI prepared as in a) with an alkali metal hydroxide solution such as NaOH or LiOH and subsequent acid treatment to give the compound of the formula I, or reacting said ester by treatment with mineral acid such as hydrochloric acid to give the carboxylic acid of the formula VII,

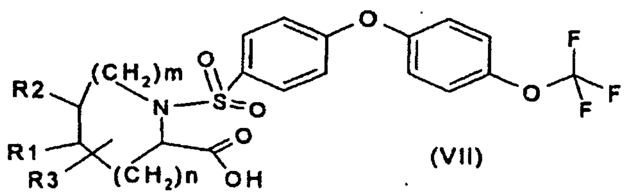

(VII)

and subsequently converting the latter into the hydroxamic acid, in which X = NH-OH, of the formula I,
c) fractionating a compound of the formula I which has been prepared by process a) or b) and which, because of its chemical structure, occurs in enantiomeric forms into the pure enantiomers by salt formation with enantiopure acids or bases, chromatography on chiral stationary phases or derivatization using chiral enantiopure compounds such as amino acids, separation of the diastereomers obtained in this way, and elimination of the chiral auxiliary groups, or
d) either isolating the compound of the formula I which has been prepared by processes b) or c) in free form or, in the case where acidic or basic groups are present, converting it into physiologically tolerated salts.

**6.** A compound of the formula III

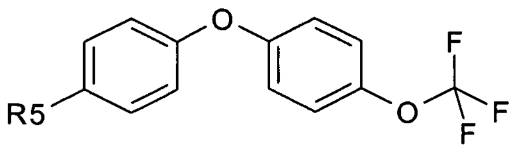

(III)

in which R5 is hydrogen atom, Li, Mg, S-$CH_3$, Cl, Br, I, Si-$(CH_3)_3$, $SO_2$-Cl, $SO_2$-Br, $SO_2$-Y, in which Y is a radical which can easily be eliminated, such as an active ester O-$R_y$, where Ry is ortho- or para-nitrophenyl, 2,4-dinitrophenyl, or pentafluorophenyl, or Y is a heterocycle such as imidazole, benzimidazole or benzotriazole, in which case the linkage takes place via the nitrogen of the heterocycle.

**7.** A process for preparing the compound of the formula III as claimed in claim 6, which comprises

a) converting the compound of the formula VIII

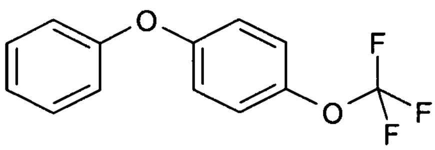

(VIII)

by reaction with chlorosulfonic acid into the corresponding arylsulfonic acid or, on use of an excess of chlorosulfonic acid, directly into the arylsulfonyl chloride, or
b) converting the compound of the formula IX,

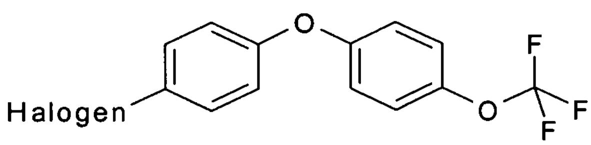

(IX)

in which halogen is Cl, Br or I, with alkyllithium such as n-BuLi into the lithiated aryls and subsequently converting the latter where appropriate by reaction with $SO_3$ amine adducts such as trimethylamine into the corresponding sulfonic acids.

**8.** A medicament which has an effective content of at least one compound of the formula I as claimed in one or more of claims 1 to 4 together with a pharmaceutically suitable and physiologically tolerated carrier, additive and/or other active substances and excipients.

**9.** The use of the compound of the formula I as claimed in one or more of claims 1 to 4 for producing a medicament for the selective prophylaxis and therapy of all disorders in the progression of which an enhanced activity of metalloproteinases are involved, such as degenerative joint disorders such as osteoarthroses, spondyloses, chondrolysis after joint trauma or prolonged joint immobilization after meniscus or patellar injuries or ligament tears or connective tissue disorders such as collagenoses, periodontal disorders, wound-healing disturbances and chronic disorders of the locomotor system such as inflammatory, immunologicallly or metabolism-related acute and chronic arthritides, arthropathies, myalgias and disturbances of bone metabolism or the treatment of ulceration, atherosclerosis and stenoses or the treatment of inflammations, cancers, tumor metastasis, cachexia, anorexia, heart failure and septic shock, or the prophylaxis of myocardial and cerebral infarctions

## Revendications

**1.** Composé de formule I

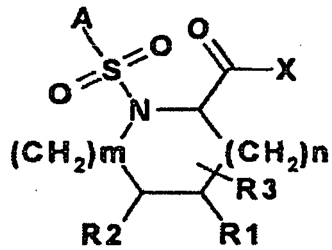

(I)

et/ou toutes formes stéréoisomères du composé de formule I et/ou tous mélanges de ces formes et tout rapport, et/ou sel physiologiquement acceptable du composé de formule I, dans laquelle

X représente -OH ou -NHOH,
A représente un radical de formule II

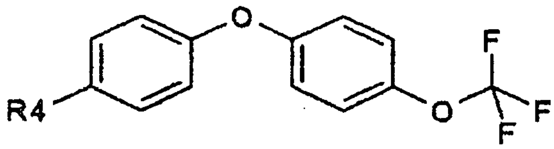

(II)

dans laquelle R4 représente la liaison covalente avec l'atome de soufre de la formule I,
R1, R2 et R3 sont identiques ou différents et représentent, chacun indépendamment,

1) un atome d'hydrogène,
2) un groupe alkyle en $C_1$-$C_6$, le groupe alkyle étant non substitué ou une ou deux fois substitué par cycloalkyle en $C_3$-$C_6$, alcénylène en $C_2$-$C_4$, alcynyle en $C_2$-$C_6$, aryle en $C_6$-$C_{14}$ ou un hétérocycle,
3) -C(O)-O-R8, où R8 représente

3)1) un atome d'hydrogène,
3)2) un groupe alkyle en $C_1$-$C_6$, le groupe alkyle étant non substitué ou substitué une ou deux fois par cycloalkyle en $C_3$-$C_6$, alcénylène en $C_2$-$C_4$, alcynyle en $C_2$-$C_6$, aryle en $C_6$-$C_{14}$ ou un hétérocycle, ou une à cinq fois par le fluor,
3)3) un groupe aryle en $C_6$-$C_{14}$ ou
3)4) un hétérocycle,

4) -O-R8, R8 ayant la signification donnée plus haut,
5) un groupe cycloalkyle en $C_3$-$C_6$,
6) un atome d'halogène,
7) -$NO_2$, ou
8) -CN, ou bien
9) R1 et R2 forment ensemble avec les atomes de carbone auxquels ils sont fixés un cycle aryle en $C_6$-$C_{14}$, le cycle étant non substitué ou une ou deux fois substitué par G,
10) R1 et R2 forment ensemble avec les atomes de carbone auxquels ils sont fixés un hétérocycle à 5, 6 ou 7 chaînons, le cycle étant non substitué ou une fois substitué par G, ou
11) R1 et R2 forment ensemble avec les atomes de carbone auxquels ils sont fixés un cycle indolyle, le cycle indolyle étant non substitué ou une ou deux fois substitué par G,

G représente

1) un atome d'hydrogène,
2) un atome d'halogène,
3) =O,
4) un groupe alkyle en $C_1$-$C_6$, le groupe alkyle étant non substitué ou une, deux ou trois fois substitué par halogène, cycloalkyle en $C_3$-$C_6$, alcénylène en $C_2$-$C_4$, alcynyle en $C_2$-$C_6$, aryle en $C_6$-$C_{14}$ ou un hétérocycle,
5) un groupe aryle en $C_6$-$C_{14}$,
6) un hétérocycle,
7) -C(O)-O-R10, où R10 représente

   a) un groupe alkyle en $C_1$-$C_6$, le groupe alkyle étant non substitué ou une ou deux fois substitué par cycloalkyle en $C_3$-$C_6$, alcénylène en $C_2$-$C_4$, alcynyle en $C_2$-$C_6$, aryle en $C_6$-$C_{14}$ ou un hétérocycle,
   b) un groupe aryle en $C_6$-$C_{14}$ ou
   c) un hétérocycle,

8) -C(S)-O-R10, où R10 est tel que défini plus haut,
9) -C(O)-NH-R11, où R11 représente

   a) un groupe alkyle en $C_1$-$C_6$, le groupe alkyle étant non substitué ou une ou deux fois substitué par cycloalkyle en $C_3$-$C_6$, aryle en $C_6$-$C_{14}$ ou un hétérocycle,
   b) un groupe aryle en $C_6$-$C_{14}$ ou
   c) un hétérocycle,

10) -C(S)-NH-R11, où R11 est tel que défini plus haut,
11) -O-R12, où R12 représente

   a) un atome d'hydrogène,
   b) un groupe alkyle en $C_1$-$C_6$, le groupe alkyle étant non substitué ou une, deux ou trois fois substitué par halogène, cycloalkyle en $C_3$-$C_6$, alcénylène en $C_2$-$C_4$, alcynyle en $C_2$-$C_6$, aryle en $C_6$-$C_{14}$ ou un hétérocycle,
   c) un groupe aryle en $C_6$-$C_{14}$,
   d) un hétérocycle,
   e) -C (O)-O-R13, où R13 représente

      e)1) un groupe alkyle en $C_1$-$C_6$, le groupe alkyle étant non substitué ou une ou deux fois substitué par cycloalkyle en $C_3$-$C_6$, alcénylène en $C_2$-$C_4$, alcynyle en $C_2$-$C_6$, aryle en $C_6$-$C_{14}$ ou un hétérocycle,
      e)2) un groupe aryle en $C_6$-$C_{14}$ ou
      e)3) un hétérocycle,

   f) -C(S)-O-R13, où R13 est tel que défini plus haut,
   g) -C(O)-NH-R14, où R14 représente

      g)1) un groupe alkyle en $C_1$-$C_6$, le groupe alkyle étant non substitué ou une ou deux fois substitué par cycloalkyle en $C_3$-$C_6$, alcénylène en $C_2$-$C_4$, alcynyle en $C_2$-$C_6$, aryle en $C_6$-$C_{14}$ ou un hétéro-

cycle,
g)2) un groupe aryle en $C_6$-$C_{14}$ ou
g)3) un hétérocycle,

h) -C(S)-NH-R14, où R14 est tel que défini plus haut,

12) -C(O)-R10, où R10 est tel que défini plus haut,
13) -S(O)p-R12, où R12 est tel que défini plus haut et p représente les nombres entiers zéro, 1 ou 2,
14) -$NO_2$,
15) -CN,
16) -N(R15)-R12, où R15 représente

16)1) un atome d'hydrogène,
16)2) un groupe alkyle en $C_1$-$C_6$,
16)3) un groupe -$SO_2$-alkyle($C_1$-$C_6$), le groupe alkyle étant non substitué ou substitué une ou deux fois par cycloalkyle en $C_3$-$C_6$, alcénylène en $C_2$-$C_4$, alcynyle en $C_2$-$C_6$, aryle en $C_6$-$C_{14}$ ou un hétérocycle, et R12 étant tel que défini plus haut, ou

17) -$SO_2$-N(R12)-R16, où R12 est tel que défini plus haut et R16 représente

17)1) un atome d'hydrogène,
17)2) un groupe alkyle en $C_1$-$C_6$, le groupe alkyle étant non substitué ou substitué une ou deux fois par cycloalkyle en $C_3$-$C_6$, alcénylène en $C_2$-$C_4$, alcynyle en $C_2$-$C_6$, aryle en $C_6$-$C_{14}$ ou un hétérocycle,
17)3) -C(O)-O-R8, où R8 a la signification donnée plus haut,
17)4) -O-R8, où R8 a la signification donnée plus haut, ou
17)5) un groupe cycloalkyle en $C_3$-$C_6$, et

m et n sont identiques ou différents et représentent les nombres zéro, 1, 2 ou 3, étant entendu que la somme de m et n a la valeur zéro, 1, 2 ou 3.

**2.** Composé de formule I selon la revendication 1, dans lequel

X représente -OH ou -NHOH,
A représente un radical de formule II
R1 et R2 forment ensemble avec les atomes de carbone auxquels ils sont fixés

a) un cycle aryle en $C_6$-$C_{14}$, aryle signifiant un radical de la série phényle, naphtyle, 1-naphtyle, 2-napthyle, anthryle ou fluorényle et étant non substitué ou une ou deux fois substitué par G, ou
b) un hétérocycle à 5, 6 ou 7 chaînons, l'hétérocycle étant un radical de la série furanne, imidazole, isoxazole, oxazole, pyrazine, pyrazole, pyridazine, pyridine, pyrimidine, thiazole ou thiophène et étant non substitué ou une fois substitué par G, ou
c) un cycle indolyle, le cycle indolyle étant non substitué ou une ou deux fois substitué par G,

G représente

1) un atome d'hydrogène,
2) un atome de fluor, chlore, brome ou iode,
3) =O,
4) un groupe alkyle en $C_1$-$C_6$, le groupe alkyle étant non substitué ou une, deux ou trois fois substitué par halogène, cycloalkyle en $C_3$-$C_6$, alcynyle en $C_2$-$C_6$, aryle en $C_6$-$C_{14}$, aryle signifiant phényle ou naphtyle, ou un hétérocycle, l'hétérocycle étant un radical choisi parmi les radicaux acridinyle, azépinyle, azétidinyle, aziridinyle, benzimidazalinyle, benzimidazolyle, benzofurannyle, benzothiofurannyle, benzothiophényle, benzoxazolyle, benzothiazolyle, benzotriazolyle, benzotétrazolyle, benzisoxazolyle, benzisothiazolyle, carbazolyle, 4aH-carbazolyle, carbolinyle, quinazolinyle, quinolinyle, 4H-quinolizinyle, quinoxalinyle, quinuclidinyle, chromanyle, chroményle, cinnolinyle, décahydroquinolinyle, dibenzofurannyle, dibenzothiophényle, dihydrofurann[2,3-b]-tétrahydrofurannyle, dihydrofurannyle, dioxolyle, dioxannyle, 2H,6H-1,5,2-dithiazinyle, furannyle, furazannyle, imidazolidinyle, imidazolinyle, imidazolyle, 1H-indazolyle, indolinyle, indolizinyle,

indolyle, 3H-indolyle, isobenzofurannyle, isochromanyle, iso-indazolyle, isoindolinyle, Iso-indolyle, isoquinolinyle (benzimidazolyle), isothiazolidinyle, 2-isothiazolinyle, isothiazolyle, isoxazolyle, isoxazolidinyle, 2-isoxazolinyle, morpholinyle, naphtyridinyle, octahydro-isoquinolinyle, oxadiazolyle, 1,2,3-oxadiazolyle; 1,2,4-oxadiazolyle, 1,2,5-oxadiazolyle, 1,3,4-oxadiazolyle, oxazolidinyle, oxazolyle, oxazolidinyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyrannyle, pyrazinyle, pyrazolidinyle, pyrazolinyle, pyrazolyle, pyridazinyle, pyrido-oxazolyle, pyrido-imidazolyle, pyridothiazolyle, pyridothiophényle, pyridinyle, pyridyle, pyrimidinyle, pyrrolidinyle, pyrrolinyle, 2H-pyrrolyle, pyrrolyle, tétrahydrofurannyle, tétrahydro-isoquinolinyle, tétrahydroquinolinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénothiazolyle, thiéno-oxazolyle, thiéno-imidazolyle, thiomorpholinyle, thiophényle, triazinyle, 1,2,3-triazolyle, 1,2,4-triazolyle, 1,2,5-triaz.olyle, 1,3,4-triazolyle et xanthényle.

5) un groupe aryle en $C_6$-$C_{14}$, aryle représentant le groupe phényle ou naphtyle,

6) un hétérocycle, l'hétérocycle étant tel que défini plus haut,

7) -C(0)-O-R10, où R10 représente

a) un groupe alkyle en $C_1$-$C_6$, le groupe alkyle étant non substitué ou une ou deux fois substitué par cycloalkyle en $C_3$-$C_6$, alcynyle en $C_2$-$C_6$, aryle en $C_6$-$C_{14}$ ou un hétérocycle, aryle signifiant phényle ou naphtyle et l'hétérocycle étant tel que défini plus haut,

b) un groupe aryle en $C_6$-$C_{14}$, aryle signifiant phényle ou naphtyle, ou

c) un hétérocycle, l'hétérocycle étant tel que défini plus haut,

8) -C(S)-O-R10, où R10 est tel que défini plus haut,

9) -C(O)-NH-R11, où R11 représente

a) un groupe alkyle en $C_1$-$C_6$, le groupe alkyle étant non substitué ou une ou deux fois substitué par cycloalkyle en $C_3$-$C_6$, aryle en $C_6$-$C_{14}$ ou un hétérocycle, aryle signifiant phényle ou naphtyle,

b) un groupe aryle en $C_6$-$C_{14}$, aryle signifiant phényle ou naphtyle, ou

c) un hétérocycle, l'hétérocycle étant tel que défini plus haut,

10) -C(S)-NH-R11, où R11 est tel que défini plus haut,

11) -O-R12, où R12 représente

a) un atome d'hydrogène,

b) un groupe alkyle en $C_1$-$C_6$, le groupe alkyle étant non substitué ou une, deux ou trois fois substitué par halogène, cycloalkyle en $C_3$-$C_6$, alcynyle en $C_2$-$C_6$, aryle en $C_6$-$C_{14}$ ou un hétérocycle, aryle signifiant phényle ou naphtyle et l'hétérocycle étant tel que défini plus haut,

c) un groupe aryle en $C_6$-$C_{14}$, aryle signifiant phényle ou naphtyle,

d) un hétérocycle, l'hétérocycle étant tel que défini plus haut,

e) -C(O)-O-R13, où R13 représente

e)1) un groupe alkyle en $C_1$-$C_6$, le groupe alkyle étant non substitué ou une ou deux fois substitué par cycloalkyle en $C_3$-$C_6$, alcynyle en $C_2$-$C_6$, aryle en $C_6$-$C_{14}$ ou un hétérocycle, aryle signifiant phényle ou naphtyle et l'hétérocycle étant tel que défini plus haut,

e)2) un groupe aryle en $C_6$-$C_{14}$, aryle signifiant phényle ou naphtyle, ou

e)3) un hétérocycle, l'hétérocycle étant tel que défini plus haut,

f) -C(S)-O-R13, où R13 est tel que défini plus haut,

g) -C(O)-NH-R14, où R14 représente

g)1) un groupe alkyle en $C_1$-$C_6$, le groupe alkyle étant non substitué ou une ou deux fois substitué par cycloalkyle en $C_3$-$C_6$, alcynyle en $C_2$-$C_6$, aryle en $C_6$-$C_{14}$ ou un hétérocycle, aryle signifiant phényle ou naphtyle et l'hétérocycle étant tel que défini plus haut,

g) 2) un groupe aryle en $C_6$-$C_{14}$, aryle signifiant phényle ou naphtyle, ou

g)3) un hétérocycle, l'hétérocycle étant tel que défini plus haut, ou

h) -C(S)-NH-R14, où R14 est tel que défini plus haut,

12) -(C(O)-R10, où R10 est tel que défini plus haut,
13) $-S(O)_p$-R12, où R12 est tel que défini plus haut et p représente les nombres entiers zéro, 1 ou 2,
14) $-NO_2$ ,
15) -CN ou
16) -N(R15)-R12, où R15 représente

16)1) un atome d'hydrogène,
16)2) un groupe alkyle en $C_1$-$C_6$,
16)3) un groupe $-SO_2$-alkyle($C_1$-$C_6$), le groupe alkyle étant non substitué ou substitué une ou deux fois par cycloalkyle en $C_3$-$C_6$, alcynyle en $C_2$-$C_6$, aryle en $C_6$-$C_{14}$ ou un hétérocycle, aryle signifiant phényle ou naphtyle et l'hétérocycle étant tel que défini plus haut, et R12 étant tel que défini plus haut, ou

17) $-SO_2$-N (R12) -R16, où R12 est tel que défini plus haut et R16 représente

17)1) un atome d'hydrogène,
17)2) un groupe alkyle en $C_1$-$C_6$, le groupe alkyle étant non substitué ou substitué une ou deux fois par cycloalkyle en $C_3$-$C_6$, alcynyle en $C_2$-$C_6$, aryle en $C_6$-$C_{14}$ ou un hétérocycle, aryle signifiant phényle ou naphtyle et l'hétérocycle étant tel que défini plus haut,
17)3) -C(O)-O-R8, où R8 a la signification donnée plus haut,
17)4) -O-R8, où R8 a la signification donnée plus haut, ou
17)5) un groupe cycloalkyle en $C_3$-$C_6$, et

m représente le nombre 1 ou 2 et n représente le nombre zéro,
m représente le nombre 1 et n représente le nombre deux ou
m et n sont identiques et représentent chacun le nombre 1.

**3.** Composé de formule I selon la revendication 1 ou 2, dans lequel
X représente -OH ou -NHOH,
A représente un radical de formule II
m représente le nombre 1 et n représente le nombre deux ou
m et n sont identiques et représentent chacun le nombre 1, and
R1 et R2 forment ensemble avec les atomes de carbone auxquels ils sont fixés un radical phényle ou tétrahydrofurannyle.

**4.** Composé de formule I selon une ou plusieurs des revendications 1 à 3, **caractérisé en ce que** le composé est
le 2-[4-(4-trifluorométhoxy-phénoxy)-benzènesulfonyl]-1,2,3,4-tétrahydro-isoquinoléinehydroxy-carboxamide,
l'acide 5-[4-(4-trifluorométhoxy-phénoxy)-benzènesulfonyl]-octahydro-furo[3,2-c]pyridine-4-carboxylique ou
le 5-[4-(4-trifluorométhoxy-phénoxy)-benzènesulfonyl]-octahydro-furo[3,2-c]pyridine-4-carboxamide.

**5.** Procédé pour la préparation du composé de formule I selon une ou plusieurs des revendications 1 à 4, **caractérisé en ce que**

a) on convertit un composé de formule III

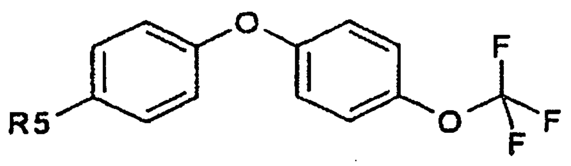

(III)

dans laquelle R5 représente un atome d'hydrogène, $NH_2$, Li, Mg, SH, $S-CH_3$, Cl, Br, I ou $Si-(CH_3)_3$, en un composé de formule IV,

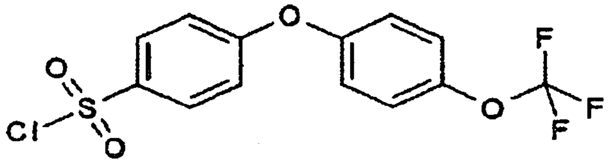

(IV)

et on fait réagir ce dernier avec un composé de formule V,

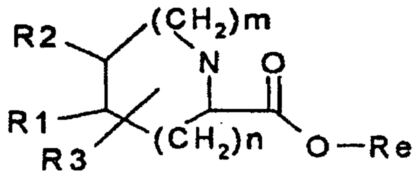

(V)

dans laquelle R1, R2, R3, m et n sont tels que définis dans la formule I et Re représente un atome d'hydrogène ou un groupe protecteur ester, en présence d'une base ou après silylation avec un agent de silylation approprié, pour obtenir un composé de formule VI,

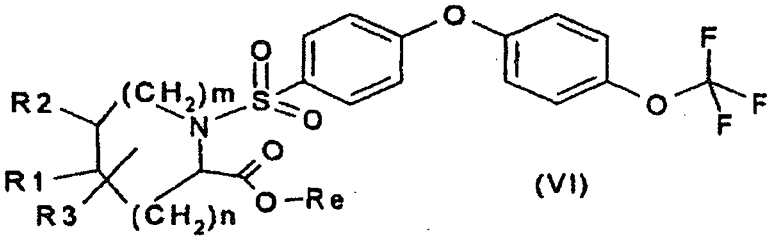

(VI)

dans laquelle R1, R2, R3, m et n sont tels que définis plus haut, et

b) dans le cas où Re = ester, on fait réagir un composé de formule VI, préparé selon a) avec une solution alcaline telle que NaOH ou LiOH, suivie d'un traitement par un acide, pour aboutir au composé de formule I, ou bien on convertit l'ester cité, par traitement par un acide minéral tel que l'acide chlorhydrique, en l'acide carboxylique de formule VII,

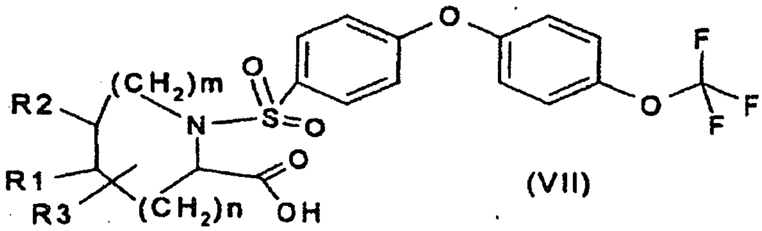

(VII)

et ensuite on convertit ce dernier en l'acide hydroxamique de formule I dans lequel X = NH-OH,

c) on dédouble en les énantiomères purs un composé de formule I, préparé selon le procédé a) ou b), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés sous formes d'énantiomères chiraux, tels que des acides aminés, séparation des diastéréoisomères ainsi obtenus et élimination des groupes auxiliaires chiraux, ou

d) soit on isole sous forme libre le composé de formule I préparé selon le procédé b) ou c), soit, dans le cas de la présence de groupes acides ou basiques on le convertit en sels physiologiquement acceptables.

**6.** Composé de formule III,

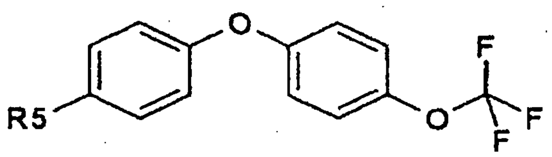

(III)

dans laquelle R5 représente un atome d'hydrogène, Li, Mg, $S-CH_3$, Cl, Br, I, $Si-(CH_3)_3$, $SO_2-Cl$, $SO_2-Br$, $SO_2-Y$, où Y représente un radical aisément séparable, tel qu'un ester actif $O-R_y$, $R_y$ représentant un groupe ortho- ou paranitrophényle, 2,4-dinitrophényle ou pentafluorophényle, ou Y représente un hétérocycle tel qu'imidazole, benzimidazole ou benzotriazole, la liaison s'effectuant par l'atome d'azote de l'hétérocycle.

**7.** Procédé pour la préparation du composé de formule III selon la revendication 6, **caractérisé en ce que**

a) on convertit le composé de formule VIII,

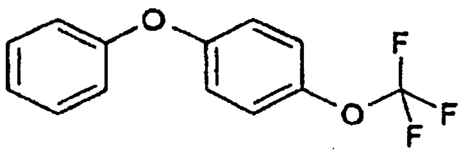

(VIII)

par la réaction avec de l'acide chlorosulfonique, en l'acide arylsulfonyle correspondant ou bien, en utilisant un excès d'acide chlorosulfonique, on le convertit directement en le chlorure d'arylsulfonyle, ou
b) on convertit le composé de formule IX,

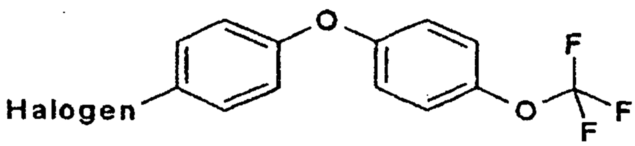

(IX)

halogène

dans laquelle "halogène" signifie C1, Br ou I, avec un alkyllithium tel que n-BuLi, et éventuellement on les convertit ensuite en les acides sulfoniques correspondants par la réaction avec des adduits $SO_3$-amine tels que la triméthylamine.

**8.** Médicament, **caractérisé par** une teneur efficace en au moins un composé de formule I selon une ou plusieurs des revendications 1 à 4, conjointement avec une substance véhicule, un additif, pharmaceutiquement appropriés et physiologiquement acceptables et/ou d'autres adjuvants et principes actifs.

**9.** Utilisation du composé de formule I selon une ou plusieurs des revendications 1 à 4 pour la fabrication d'un médicament destiné à la prophylaxie et la thérapie sélectives de toutes les maladies à l'évolution desquelles participe une activité accrue des métalloprotéinases, telles que des arthropathies dégénératives telles que des ostéo-arthroses, des spondyloses, la chondroporose après traumatisme articulaire ou relativement longue immobilisation d'articulations après blessures du ménisque ou de la rotule ou déchirures des ligaments, ou des maladies du tissu conjonctif telles que les collagénoses, les maladies périodontaires, les troubles de la cicatrisation et des maladies chroniques de l'appareil moteur, telles que les arthrites, arthropathies, myalgies, aiguës et chroniques d'origine inflammatoire, immunologique ou métabolique, et des troubles du métabolisme du tissu osseux ou le traitement de l'ulcération, de l'athérosclérose ou le traitement d'inflammations, de maladies cancéreuses, de la formation de métastases tumorales, de la cachexie, de l'anorexie, de l'insuffisance cardiaque et du choc septique ou la prophylaxie d'infarctus du myocarde et cérébraux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9718194 A
- WO 03016248 A
- US 5430023 A
- US 5726159 A
- EP 643073 A
- US 4902695 A
- WO 2003041641 A
- DE 3322530
- DE 3211676
- US 4782094 A
- EP 0257415 A


### In der Beschreibung aufgeführte Nicht-Patentliteratur


- *Tetrahedron,* 1997, vol. 53, 14773-92
- *Tetrahedron,* 1992, vol. 48, 4659-76
- *Synthesen in Tetrahedron,* 1999, vol. 55, 8025
- *Tetrahedron Lett.,* 1983, vol. 24, 5339
- **T.H. GREENE ; P. G. M. WUTS.** Protective Groups in Organic Synthesis. Wiley-Interscience, 1999
- *J. Org. Chem.,* 1980, vol. 45, 547
- *Indian J. Chem.,* 1986, vol. 25, 1273
- *J. Org. Chem.,* 1992, vol. 57, 190
- *Nippon Kagaku Kaishi,* 1976, vol. 4, 631-6
- *Tetrahedron Lett.,* 1987, vol. 28, 2115
- *Org. Synth. I,* 1941, vol. 8 und 85
- *Recl. Trav. Chim. Pays-Bas,* 1988, vol. 107, 418
- *Bull. Soc. Chim. Fr.,* 1980, 195
- *J. Am. Chem. Soc.,* 1949, vol. 71, 1593
- *Recl. Trav. Chim. Pays-Bas,* 1992, vol. 111, 215
- *J. Prakt. Chem.,* 1963, vol. 22, 290
- *J. Prakt. Chem.,* 1916, vol. 93, 183
- *Bioorg. Med. Chem. Lett.,* 1999, vol. 9, 1251
- *J. Med. Chem.,* 1984, vol. 27, 1740
- *Org. Synth.,* 1981, vol. 60, 121
- *Chem. Ber.,* 1957, vol. 90, 841
- *Org. Synth.,* 1990, vol. VII, 508
- *J. Org. Chem.,* 1996, vol. 61, 1530
- *J. Chem. Soc., Perkin I,* 1996, vol. 13, 1583
- *Synthesis,* 1986, 852
- *Chem. Ber.,* 1995, vol. 128, 575
- *Chem. Lett.,* 1992, vol. 8, 1483
- *Synthesis,* 1998, vol. 11, 1593
- *Org. Lett.,* 2004, vol. 6, 913
- *Tetrahedron,* 2000, vol. 56, 5045
- *Heterocycl. Chem.,* vol. 36, 1453
- *Org. Lett.,* 2003, vol. 5, 3799
- *Synlett,* 2003, vol. 11, 1734
- *Organic Chemistry,* 2002, 1-8
- *J. Am. Chem. Soc.,* 1997, vol. 119, 10539
- **YE et al.** *Biochemistry,* 1992, vol. 31, 11231-11235